(19) Europäisches Patentamt
European Patent Office
Office européen des brevets

(11) EP 2 491 915 B1

(12) **EUROPEAN PATENT SPECIFICATION**

(45) Date of publication and mention of the grant of the patent:
**03.07.2019 Bulletin 2019/27**

(21) Application number: **10825090.3**

(22) Date of filing: **22.10.2010**

(51) Int Cl.:
***A61K 6/00*** (2006.01) ***A61K 6/06*** (2006.01)
***A61K 6/08*** (2006.01) ***A61K 6/083*** (2006.01)

(86) International application number:
**PCT/JP2010/069231**

(87) International publication number:
**WO 2011/049244 (28.04.2011 Gazette 2011/17)**

(54) **EASILY REMOVABLE CURABLE COMPOSITION FOR DENTAL USE**

LEICHT ENTFERNBARE HÄRTBARE ZUSAMMENSETZUNG ZUR ZAHNÄRZTLICHEN VERWENDUNG

COMPOSITION DURCISSABLE POUVANT ÊTRE FACILEMENT RETIRÉE POUR UTILISATION DENTAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.10.2009 JP 2009244439**

(43) Date of publication of application:
**29.08.2012 Bulletin 2012/35**

(73) Proprietor: **SUN MEDICAL CO., LTD.**
**Shiga 524-0044 (JP)**

(72) Inventors:
- **ORI Tatsuya**
  **Moriyama-shi**
  **SHIGA 524-0044 (JP)**
- **NISHITANI Haruka**
  **Moriyama-shi**
  **SHIGA 524-0044 (JP)**
- **IWASAKI Sayuri**
  **Moriyama-shi**
  **SHIGA 524-0044 (JP)**
- **YAMAMOTO Yuya**
  **Moriyama-shi**
  **SHIGA 524-0044 (JP)**
- **NISHIMOTO Chidzuru**
  **Moriyama-shi**
  **SHIGA 524-0044 (JP)**

(74) Representative: **Beckmann, Claus et al**
**Kraus & Weisert**
**Patentanwälte PartGmbB**
**Thomas-Wimmer-Ring 15**
**80539 München (DE)**

(56) References cited:
**EP-A1- 1 695 685** **WO-A1-2007/018220**
**JP-A- 2000 143 432** **JP-A- 2003 512 403**
**JP-A- 2008 024 668** **US-A1- 2002 198 284**
**US-A1- 2009 137 697** **US-A1- 2009 227 699**
**US-B1- 6 472 454**

- **DATABASE WPI Week 200035 Thomson Scientific, London, GB; AN 2000-407068 XP002721762, & JP 2000 143432 A (SAN MEDICAL KK) 23 May 2000 (2000-05-23)**
- **DATABASE WPI Week 200835 Thomson Scientific, London, GB; AN 2008-F12329 XP002721763, & JP 2008 024668 A (TOKUYAMA CORP) 7 February 2008 (2008-02-07)**
- **DATABASE WPI Week 200553 Thomson Scientific, London, GB; AN 2005-515004 XP002721764, & JP 2005 179282 A (KURARE MEDICAL KK) 7 July 2005 (2005-07-07)**
- **DATABASE WPI Week 200882 Thomson Scientific, London, GB; AN 2008-O11205 XP002721765, & JP 2008 285645 A (TOKUYAMA DENTAL KK) 27 November 2008 (2008-11-27)**
- **DATABASE WPI Week 200903 Thomson Scientific, London, GB; AN 2009-A62016 XP002721766, & JP 2008 308418 A (SAN MEDICAL KK) 25 December 2008 (2008-12-25)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 491 915 B1

- DATABASE WPI Week 198809 Thomson Scientific, London, GB; AN 1988-061262 XP002721767, & JP S63 17816 A (NIPPON OILS & FATS CO LTD) 25 January 1988 (1988-01-25)

## Description

TECHNICAL FIELD

The present invention relates to a dental curable composition. More specifically, it relates to an easily removable dental curable composition which can be widely used as a dental bonding material, cement, root canal filling material, lining material, temporary sealing material, temporary luting material or another repair material, has excellent marginal sealability and is easily removed when it must be removed.

BACKGROUND ART

As bonding materials and cements which are adhesive materials for the repair of a missing part of a tooth, there are proposed various compositions which need to or do not need to be pretreated in order to decalcify the tooth surface with an acid aqueous solution or a primer. Basically, the operation of pretreating the tooth surface is very effective in removing a smear layer formed on the cut tooth surface or contaminants adhered to the tooth surface so as to obtain stable adhesion to the dentine. However, it is very difficult for an operator to carry out the pretreatment of the tooth surface in a narrow oral cavity within a limited period of time without fail. Even when the operator can pretreat the tooth surface, if the adhesive material cannot be polymerized on the moist tooth surface, that is, in a wet condition, it is impossible to achieve stable adhesion to the dentine. Particularly when polymerization is greatly impeded at the interface with the dentine, there occur problems with adhesion durability and marginal sealability, thereby causing secondary dental caries in the end.

As problems with the repair of dental caries in the cervical portion of a tooth, prosthetic repair and the treatment of a root canal, the following special properties are included in the properties required for repair materials, in addition to the above properties.

In the case of the repair of dental caries in the cervical portion of a tooth, as an excessive load is applied to the cervical portion, the repair material must have high adhesion durability and marginal sealability as a matter of course, and also it is important that the repair material should have high mechanical properties, especially flexibility and abrasion resistance. It is not an exaggeration to say that being excellent in all of these properties affects a prognosis after treatment. For the repair of this application site, a dental composition comprising methyl methacrylate, polymethyl methacrylate and trialkyl boron as a polymerization initiator is preferred as it has high adhesion performance and elastic property. However, as the pretreatment of the tooth surface is essential, the operation becomes complicated and technic sensitive, and as the dental composition does not comprise an inorganic filler in a sufficient amount, it is hard to say that the dental composition has satisfactory abrasion resistance, and the dental composition may mar the aesthetic property of its margin with the dentine.

In the case of prosthetic repair, a method in which a temporary sealing material is provisionally filled in a missing part or a cavity after the removal of dental caries for a certain period of time until a prosthetic appliance manufactured outside the oral cavity such as an inlay or a crown is bonded to the missing part after the dental caries is removed is carried out. However, it is not possible to avoid the invasion of bacteria into the oral cavity by bonding with a temporary sealing material, and the bonded surface is contaminated by the invasion of saliva or food dissolved in saliva, whereby a bonding problem may occur thereafter.

Also in the case of the treatment of a root canal, a method in which a gutta-percha point or a combination of the gutta-percha point and a root canal filling material used to fill the space between a root canal wall and a gutta-percha point, called "sealer", is filled into the root canal having no dental pulp is generally carried out. As the sealer, there are proposed an epoxy-based resin, a resin-based curable composition and a glass ionomer cement which comprises polyacrylic acid and alumino silicate glass as the main components and is said to be cured by an acid-base reaction. Since all of them have no adhesion to the root canal wall (dentine) and to the gutta-percha point, even when the inside of the root canal is completely sterilized before the root canal is filled, it is fairly possible that bacteria invade the inside of the root canal again from the crown side or the apical side, whereby a problem may occur in the prognosis after treatment and retreatment may be necessary.

DISCLOSURE OF THE INVENTION

To solve the above problems, it is an object of the present invention to provide a dental curable composition for use as a bonding material or a cement which has not only excellent marginal sealing characteristic due to a high interfacial polymerization characteristic effect for a moist tooth surface without requiring a pretreatment but also preferred flexibility and abrasion resistance for the repair of dental caries in the cervical portion of a tooth and also as a lining material which has excellent marginal sealability and blocks off a chemical stimulus and a temperature stimulus to the dental pulp from a repair material. It is another object of the present invention to provide a dental curable composition

which does not require a pretreatment, has excellent marginal sealability until it is removed and is easily removed when it must be removed as a root canal filling material which must be removed when retreatment is necessary and as a temporary sealing material or a temporary luting material which can be removed when a prosthetic application is attached.

The inventors of the present invention conducted intensive studies to attain the above objects in view of the above situation and found a dental curable composition whose polymerization is not impeded even in a wet environment and in the presence of oxygen and which has excellent marginal sealability due to its high interfacial polymerization characteristic for a tooth not subjected to a surface treatment, can adjust the mechanical properties of a cured product obtained through polymerization by controlling the content of a soft resin material, and enables the provision of flexibility and the control of removability. The present invention was accomplished based on this finding.

That is, the dental curable composition of the present invention comprises (A) a polymerizable monomer, (B) an organic amine-based polymerization initiator and (C) a soft resin material as defined in the appended claims.

As described above, the dental curable composition of the present invention has excellent adhesion to the moist tooth surface as a matter of course and also forms a layer structure that the both components of the composition are impregnated into the dentine and interpenetrated with each other three-dimensionally. Particularly when the composition is used alone as a root canal filling material, the composition infiltrates into the dentine and is polymerized to form a mutually infiltrated polymer network layer (to be referred to as "resin impregnated layer") . When the composition is used as a root canal sealer in combination with a gutta-percha point, the composition infiltrates into not only the dentine but also the gutta-percha point to be polymerized, thereby forming a three-dimensionally interpenetrated layer structure. Therefore, more excellent sealability is obtained.

## BRIEF DESCRIPTION OF THE DRAWINGS

Fig. 1 shows an SEM image of a joint interface between a gutta-percha point and the composition; and
Fig. 2 is a graph of EDS analysis (joint interface between the gutta-percha point and the composition).

## BEST MODE FOR CARRYING OUT THE INVENTION

The dental curable composition of the present invention comprises (A) a polymerizable monomer, (B) an organic amine-based polymerization initiator and (C) a soft resin material as defined in the appended claims.

The dental curable composition of the present invention comprises (A) a polymerizable monomer. The polymerizable monomer is not particularly limited if it is a monomer which is polymerized by a radical polymerization initiator. A monomer having a (meth)acryloyl group, styryl group, vinyl group or allyl group as the polymerizable group is preferably used.

In the present invention, "(meth)acrylate" is a collective term for both acrylate and methacrylate, and "(meth) acrylic acid" and "(meth)acryloyl group" should be understood likewise.

The polymerizable monomer (A) used in the present invention should have at least one polymerizable group in one molecule. In the present invention, the polymerizable group in the polymerizable monomer (A) in the following description is synonymous with this.

In the present invention, a monofunctional polymerizable monomer having one polymerizable group in one molecule, a bifunctional polymerizable monomer having two polymerizable groups, or a polyfunctional polymerizable monomer having 3 or more polymerizable groups (such as polyfunctional (meth) acrylate) may be used as the polymerizable monomer (A) and is suitably selected according to use purpose.

Examples of the monofunctional polymerizable monomer (i) include linear or branched alkyl (meth)acrylates such as methyl (meth) acrylate, ethyl (meth)acrylate, propyl (meth)acrylate, isopropyl meth(acrylate), butyl (meth)acrylate, pentyl(meth)acrylate and isopentyl (meth)acrylate; hetereocyclic(meth)acrylates containing an oxygen atom and so on such as glycidyl(meth)acrylate and tetrahydrofurfuryl(meth)acrylate; (meth)acrylates containing a hydroxyl group such as 2-hydroxyethyl (meth)acrylate, 2- or, 3-hydroxypropyl(meth)acrylate, 4-hydroxybutyl(meth)acrylate, 5-hydroxypentyl (meth)acrylate, 6-hydroxyhexyl(meth)acrylate, 10-hydroxydecyl(meth)acrylate, 1,2- or 1,3- or 2,3-dihydroxypropane(meth)acrylates, diethylene glycol mono(meth)acrylate, triethylene glycol mono(meth)acrylate, pentaethylene glycol mono(meth)acrylate, polyethylene glycol mono(meth)acrylate and dipropylene glycol mono(meth)acrylate; (meth)acrylamides containing a hydroxyl group such as methylol (meth)acrylamide, N-(meth)acryloyl-2,3-dihydroxypropylamine and N-(meth)acryloyl-1,3-dihydroxypropylamine; adducts of GMA with an aliphatic or aromatic polyol (including phenol) such as 2-hydroxy-3-phenoxypropyl(meth)acrylate (HPPM in the case of methacrylate), 2-hydroxy-3-naphthoxypropyl (meth)acrylate (HNPM in the case of methacrylate) and addition reaction product (Bis-GMA in the case of methacrylate) of 1 mol of bisphenol A with 2 mols of glycidyl (meth)acrylate (GMA in the case of methacrylate); hydroxyalkyl (meth)acrylates having a halogen such as chlorine, such as 3-chloro-2-hydroxypropyl(meth)acrylate; and alkoxypolyethylene

glycol(meth)acrylates such as ethylene glycol mono(meth)acrylate, diethylene glycol mono(meth)acrylate, triethylene glycol mono (meth) acrylate, polyethylene glycol mono(meth)acrylate, methoxydiethylene glycol mono(meth)acrylate, methoxytetraethylene glycol (meth)acrylate and methoxypolyethylene glycol (meth)acrylate.

Examples of the bifunctional polymerizable monomer (ii) include linear or branched poly- or mono-alkylene glycol di(meth)acrylates such as methylene glycol di(meth)acrylate, ethylene glycol di(meth)acrylate, diethylene glycol di(meth)acrylate, triethylene glycol di(meth)acrylate, propylene glycol di(meth)acrylate, tetraethylene glycol di(meth)acrylate, polyethylene glycol di(meth)acrylate, 1,3-butylene glycol di(meth)acrylate and neopentyl glycol di (meth)acrylate.

Examples of the polymerizable monomer having 3 or more polymerizable groups (iii) include trifunctional polymerizable monomers such as trimethylolalkane tri(meth)acrylates including trimethylolmethane tri (meth) acrylate, trimethylolethane tri (meth) acrylate and trimethylolpropane tri(meth)acrylate ($CH_3$-$CH_2$-C(-$CH_2$O-CO-CR=$CH_2$)$_3$, R: H or $CH_3$) and (meth) acrylate esters of tris(2-hydroxyethyl)isocyanurate; tetrafunctional monomers such as tetra(meth)acrylates of a polymethylolalkane or an ether thereof including pentaerythritol tetra(meth)acrylate and ditrimethylolpropane tetra(meth)acrylate (O(-$CH_2$-C(-$CH_2$O-CO-CR=$CH_2$)$_2$$CH_2$$CH_3$)$_2$, R: H or $CH_3$) ; and polymerizable monomers having 5 or more polymerizable groups such as poly(meth)acrylates of a polymethylolalkane or an ether thereof including dipentaerythritol hexa(meth)acrylate and dipentaerythritol hydroxypenta(meth)acrylate.

Polymerizable monomers having 2 or more polymerizable groups also include compounds having a methacrylate group and an acrylate group in one molecule such as triethylene glycol acrylate methacrylate, trimethylolpropane monoacrylate dimethacrylate and pentaerythritol diacrylate dimethacrylate.

Out of these polymerizable monomers, (A') a long-chain polymerizable monomer having a chain length of 19 or more atoms, preferably 19 to 300 atoms, more preferably 25 to 200 atoms, much more preferably 30 to 100 atoms is preferably used as the above component (A) to enhance the flexibility and removability of a cured product. The long-chain structure is not limited to a specific chemical structure as long as the molecular chain is sufficiently long and has flexibility. As examples of the long-chain structure, polyalkylene glycol di(meth)acrylates having at least 4 oxyalkylene recurring units (-(-(-$CH_2$-)$_p$-O-)$_n$-; p is an integer of 2 or more, and n is an integer of 4 or more) are preferably used, and polyethylene glycol di(meth)acrylate and/or polypropylene glycol di(meth)acrylate having preferably 4 to 60, more preferably 9 to 40, much more preferably 11 to 28 recurring units derived from propylene glycol and/or ethylene glycol are/is more preferably used. When the number of the above atoms or the number of recurring units is smaller than the lower limit, removal becomes difficult and when the number is larger than the upper limit, polymerization becomes incomplete disadvantageously.

As for the chain length of the above long-chain polymerizable monomer, it is assumed that the free rotation of a bond between atoms has a great influence upon flexibility. Therefore, it is considered that a double bond is not preferred. Consequently, to calculate the number of the above atoms, a numerical value obtained by subtracting the number of double bonds is preferably adopted. More specifically, an atom group (>C=C<) bonded by a double bond on the molecular chain route of interest is counted as one atom. This is because an atom bonded by a single bond on the molecular chain has two bond axes which can be freely turned whereas two carbon atoms bonded by a double bond on the molecular chain route have only two bond axes which can be freely turned in total. An aromatic ring or a condensed ring or spiro ring thereof should be counted as one atom. A 3-membered or 4-membereed ring should be counted likewise. Although a 5 or more-membered ring has the degree of freedom of conformational conversion into a chair-like or boat-like form, as it has limits, it is appropriate to count the number of atoms of the ring as a total of (1 ÷ 1/4) by adding 1/4. It is also appropriate to add 1/4 each time a 5 or more-membered ring is added as a condensed ring or spiro ring. According to this, for example, the number of atoms of a bicyclo condensed ring (perfectly hydrogen saturated naphthalene ring) is (1 + 1/4 + 1/4). It is not necessary to take into account a double bond not existent on the molecular chain like the oxygen of a carbonyl. It is preferred not to count the atom (such as hydrogen) at, the end of the molecular chain. Even when the molecular chain is slightly short, it is preferred to count the number of atoms by selecting a route in which a polymerizable double bond is existent at the end of the molecular chain.

Further, it is preferred that the long-chain polymerizable monomer (A') should have at least two polymerizable groups, and it is more preferred that the component (A') should have a polymerizable double bond at both ends of the above long-chain structure. Alternatively, it is assumed that it is important that the long-chain structure should be existent between at least two polymerizable double bonds. Probably, in a stable thermodynamic equilibrium state, it is considered that the long-chain structure in a moderately shrunk form is more predominant than in an elongated form and that this structure is still in that state even when the polymerizable double bonds at both ends form a bridge between polymerization molecules. It is assumed that when the long-chain polymerizable monomer is cured by polymerization to enter a polymerization shrinkage step, the above shrunk form changes into the elongated form, thereby contributing to the relaxation of stress by polymerization shrinkage. For example, long-chain polymerizable monomers having 4 to 60 recurring units derived from polyethylene glycol and polypropylene glycol represented by the following formulas (III) and (IV) are particularly preferred.

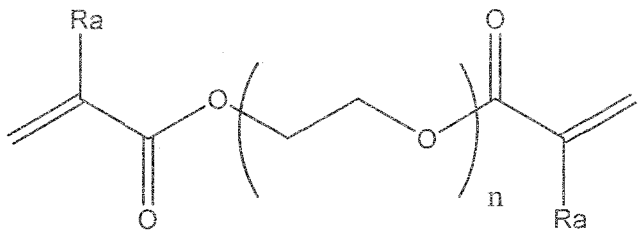

(III)

In the above formula (III), Ra is H or $CH_3$, and n is 4 to 60 .

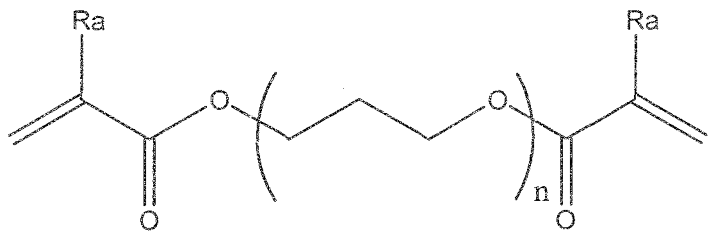

(IV)

In the above formula (IV), Ra is H or $CH_3$, and n is 4 to 60.

Besides the above monomers, a polymerizable monomer containing a hydroxyl group in the molecule, a polymerizable monomer containing a triazine ring derivative represented by the following formula (V) (isocyanurate (meth)acrylate ester) and a dipentaerythritol-based polymerizable monomer represented by the following formula (VI) are particularly preferably used. The above long-chain polymerizable monomers may be used alone or in combination.

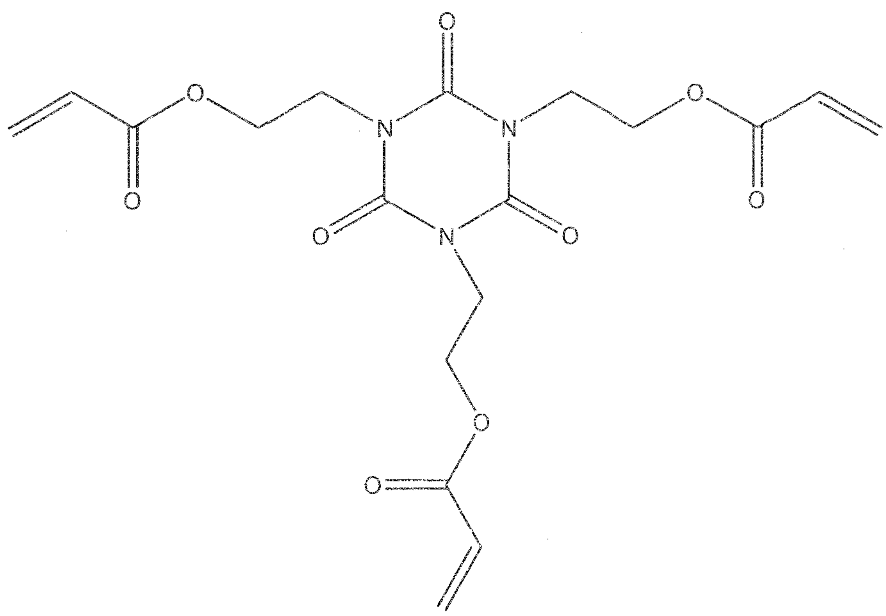

(V)

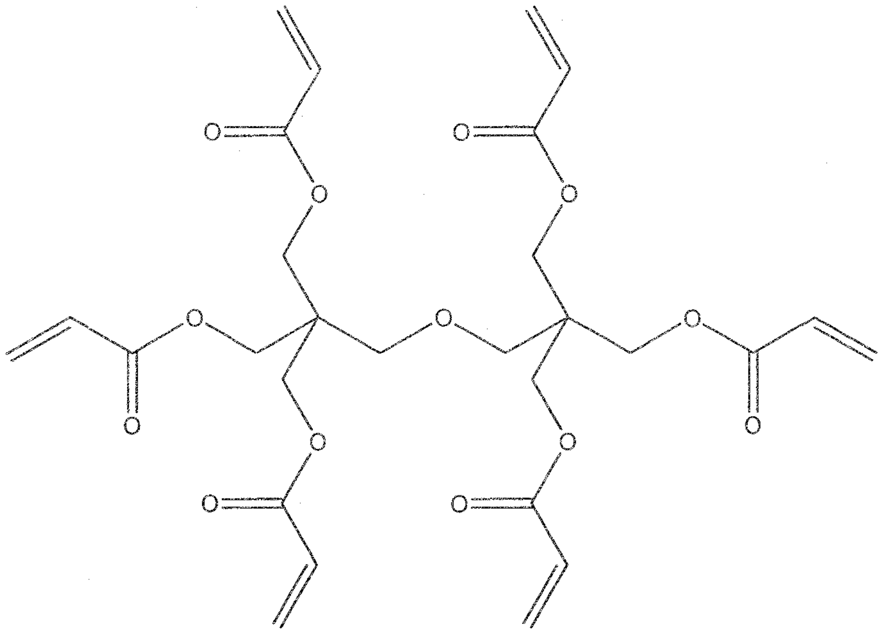

(VI)

Although the triazine ring derivative is not particularly limited, examples thereof include substitution products obtained by substituting at least one hydrogen of a triazine ring by another substituent having a polymerizable functional group and derivatives obtained by saturating at least one double bond of a triazine ring to introduce a substituent having a polymerizable functional group into a carbon or nitrogen atom constituting the ring. Derivatives obtained by saturating the double bond of the cyclic structure of the triazine ring, carbonylating a carbon atom and introducing a substituent having a polymerizable functional group into nitrogen atoms, that is, isocyanurate-based compounds having a structure represented by the following formula (VII) are preferred. In the formula (VII), $R_{17}$, $R_{18}$ and $R_{19}$ are each independently a polymerizable group, for example, a radically polymerizable unsaturated group having a (meth)acryloyl group, vinyl group or allyl group.

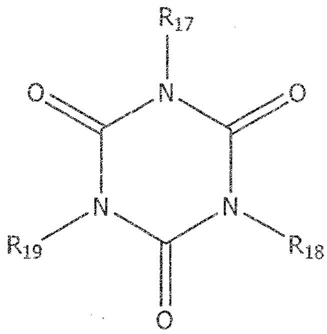

(VII)

Besides the above monomers, polymerizable monomers having an urethane bond, for example, polymerizable monomers having a diurethane di (meth) acrylate structure represented by the following formula (VIII) are also particularly preferably used. They may be used alone or in combination.

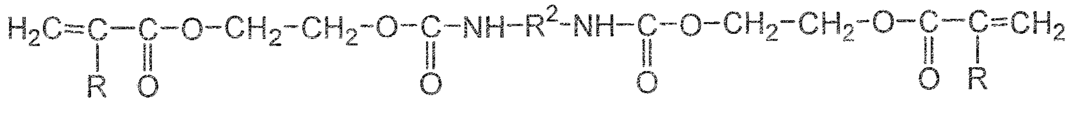

(VIII)

In the above formula, R is a hydrogen atom or methyl group, and the structure of $R^2$ may be selected from the following structures (a) to (g).

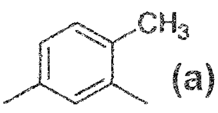 (a) 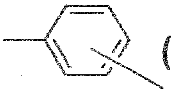 (b) 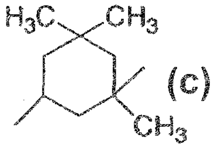 (c)

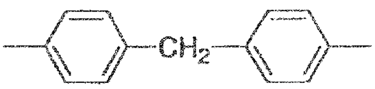 (d) 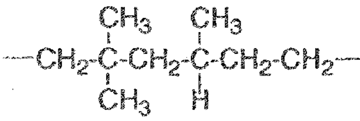 (e)

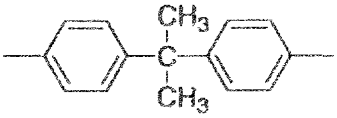 (f) 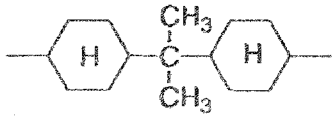 (g)

In the case of (e), 1,6-bis(methacryloxyethyloxycarbonylamino)-2,2,4-trimethylhexane (UDMA) is particularly preferred.

The tetrafunctional monomer of this urethane-based compound is, for example, a compound represented by the following formula (IX).

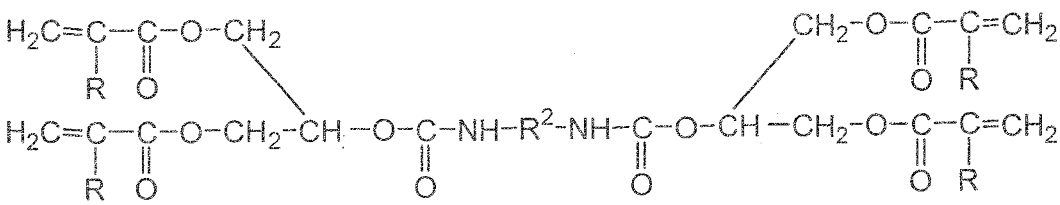

(IX)

In the above formula (IX), $R_2$ is as defined in the above formula (VIII).

These polymerizable monomers may be used alone or in combination.

Although all of the above polymerizable monomers have no acid group in the molecule, the polymerizable monomer (A) used in the present invention includes a polymerizable monomer containing an acid group in the molecule. In the present invention, a functional group which easily changes into an acid group like an acid anhydride is regarded as an acid group. Examples of the acid group in the polymerizable monomer include carboxylic acid group, phosphoric acid group, pyrophosphoric acid group, thiophosphoric acid group, sulfonic acid group, sulfinic acid group and acid anhydrides thereof. The component (A) preferably contains at least one of these acid groups.

The polymerizable monomer having at least one carboxyl group in one molecule is selected from a monocarboxylic acid, dicarboxylic acid, tricarboxylic acid, tetracarboxylic acid and derivatives thereof. Examples thereof include compounds having a carboxyl group directly bonded to a vinyl group such as (meth)acrylic acid, fumaric acid and maleic acid; compounds having an aromatic ring directly bonded to a vinyl group such as p-vinylbenzoic acid; compounds having a (meth)acryloyloxy group such as 11-(meth)acryloyloxy-1,1-undecanedicarboxylic acid (MAC-10 in the case of methacrylate); aromatic carboxylic acid compounds having a (meth)acryloyloxyalkyl group such as 1,4-di(meth)acryloyloxyethylpyromellitic acid and 6-(meth)acryloyloxyethylnaphthalene-1,2,6'-tricarboxylic acid; (hydroxyl) (meth)acryloyloxyalkyltrimellitic acid compounds and anhydrides thereof such as 4-(meth)acryloyloxymethyltrimellitic acid and anhydride thereof, 4-(meth)acryloyloxyethyltrimellitic acid (4-MET in the case of methacrylate) and anhydride thereof (4-META in the case of methacrylate), 4-(meth)acryloyloxybutyltrimellitic acid and anhydride thereof, and 4-[2-hydroxy-3-(meth)acryloyloxy]butyltrimellitic acid and anhydride thereof; compounds having carboxybenzoyloxy such as 2,3-bis(3,4-dicarboxybenzoyloxy)propyl (meth)acrylate; N- and/or O-(meth)acryloyloxyamino acids such as N,O-di-(meth)acryloyloxytyrosine, O-(meth)acryloyloxytyrosine, N-(meth)acryloyloxytyrosine and N-(meth)acryloyloxyphenylalanine; (meth)acryloylaminobenzoic acids such as N-(meth)acryloyl-p-aminobenzoic acid, N-(meth)acryloyl-O-aminobenzoic acid, N-(meth)acryloyl-5-aminosalicylic acid (5-MASA in the case of methacrylate) and N-(meth)acryloyl-4-aminosalicylic acid; and adducts such as an adduct of 2-hydroxyethyl (meth)acrylate with pyromellitic dianhydride (PMDM in the case of methacrylate), an adduct of 2-hydroxyethyl (meth)acrylate with maleic anhydride, 3,3',4,4'-benzophenone tetracarboxylic dianhydride (BTDA in the case of methacrylate) or 3,3',4,4'-biphenyltetracarboxylic dianhydride, an adduct of 2-(3,4-dicarboxybenzoyloxy)1,3-di(meth)acryloyloxy propane, N-phenylglycine or N-tolylglycine with glycidyl

(meth)acrylate, and compounds having an alcoholic hydroxyl group such as 4-[(2-hydroxy-3-(meth)acryloyloxypropyl)amino]phthalic acid and 3- or 4-[N--methyl-N-(2-hydroxy-3-(meth)acryloyloxypropyl)amino ]phthalic acid. Out of these, MAC-10, 4-MET, 4-META and 5-MESA are preferably used. These polymerizable monomers having a carboxyl group may be used alone or in combination.

Out of the polymerizable monomers which can be used as the component (A), polymerizable monomers having at least one phosphoric acid group in one molecule include (meth)acryloyloxyalkyl acid phosphate compounds such as 2-(meth)acryloyloxyethyl acid phosphate, 2- and 3-(meth)acryloyloxypropyl acid phosphate, 4-(meth)acryloyloxybutyl acid phosphate, 6-(meth)acryloyloxyhexyl acid phosphate, 8-(meth)acryloyloxyoctyl acid phosphate, 10-(meth)acryloyloxydecyl acid phosphate and 12-(meth)acryloyloxydodecyl acid phosphate; and bis{(meth)acryloyloxyalkyl}acid phosphates such as bis{2-(meth)acryloyloxyethyl}acid phosphate and bis{2- or 3-(meth)acryloyloxypropyl}acid phosphate, and (meth)acryloyloxyalkylphenyl acid phosphates having or not having a substituent on an aromatic ring such as 2-(meth)acryloyloxyethylphenyl acid phosphate and 2-(meth)acryloyloxyethyl-p-methoxyphenyl acid phosphate. The phosphoric acid group in these compounds can be substituted by a thiophosphoric acid group. Out of these, 2-(meth)acryloyloxyethylphenyl acid phosphate and 10-(meth)acryloyloxydecyl acid phosphate are preferably used. These polymerizable monomers having a phosphoric acid group may be used alone or in combination.

Out of the polymerizable monomers which can be used as the component (A), polymerizable monomers having at least one pyrophosphoric acid group in one molecule include di{(meth)acryloyloxyalkyl}pyrophosphate compounds such as di{2-(meth)acryloyloxyethyl}pyrophosphate, di{4-(meth)acryloyloxybutyl}pyrophosphate, di{6-(meth)acryloyloxyhexyl}pyrophosphate, di{8-(meth)acryloyloxyoctyl}pyrophosphate and di{10-(meth)acryloyloxydecyl}pyrophosphate. These polymerizable monomes having a pyrophosphoric acid group may be used alone or in combination.

Out of the polymerizable monomers which can be used as the component (A), polymerizable monomers having at least one sulfonic acid group in one molecule include sulfoalkyl (meth)acrylate compounds such as 2-sulfoethyl (meth) acrylate, 2- or 1-sulfo-1- or 2-propyl (meth) acrylate and 1- or 3-sulfo-2-butyl (meth)acrylate; sulfoalkyl (meth)acrylate having another substituent such as hetero atom, such as 3-bromo-2-sulfo-2-propyl (meth)acrylate and 3-methoxy-1-sulfo-2-propyl (meth)acrylate; and sulfoalkyl (meth)acrylamides having a substituent such as 1,1-dimethyl-2-sulfoethyl (meth)acrylamide. Out of these, 2-methyl-2-(meth)acrylamidepropane sulfonic acid is preferably used. These polymerizable monomers having a sulfonic acid group may be used alone or in combination.

All the above components (A) may be used alone or in combination.

The acid group of the polymerizable monomer containing an acid group in the molecule is existent in the component (A) of the present invention in an amount of preferably 0.00001 to 0.03 mol/g, more preferably 0.0001 to 0.01 mol/g, much more preferably 0.0005 to 0.006 mol/g in terms of a monovalent acid group.

When the acid group releases $1H^+$ through an acid-base neutralization reaction like a carboxyl group, it is simply equivalent to the number of mols/g of the monovalent acid group and when the acid group releases $2H^+$ through an acid-base neutralization reaction like a phosphoric acid group ($-H_2PO_4$), it is equivalent to 2 times the number of mols/g of the monovalent acid group.

When the amount of the acid group falls below the lower limit, acidity for decalcifying the tooth surface may become unsatisfactory and when the amount of the acid group exceeds the upper limit, the tooth surface may be over decalcified.

The hydroxyl group of the polymerizable monomer having an alcoholic hydroxyl group is existent in the component (A) of the present invention in an amount of preferably 0.00001 to 0.02 mol/g, more preferably 0.0001 to 0.01 mol/g, much more preferably 0.001 to 0.008 mol/g. When the amount of the hydroxyl group falls below the lower limit, hydrophobic nature may become strong and compatibility with water may lower and when the amount of the hydroxyl group exceeds the upper limit, the water resistance of the polymer may degrade.

A polymerizable polyfunctional (meth) acrylate having at least 3 ethylenically unsaturated bonds is contained in the component (A) of the present invention in an amount of preferably 1 to 70 parts by weight, more preferably 5 to 50 parts by weight, much more preferably 10 to 30 parts by weight (based on 100 parts by weight of the whole component (A)). When the amount of the polymerizable polyfunctional (meth) acrylate falls below the lower limit, the mechanical strength of the polymer may become unsatisfactory and when the amount exceeds the upper limit, the polymer may become brittle.

A polymerizable monomer having a triazine ring derivative is advantageously contained in the component (A) of the present invention in an amount of preferably 1 to 70 parts by weight, more preferably 10 to 50 parts by weight, much more preferably 20 to 40 parts by weight (based on 100 parts by weight of the whole component (A)). When the amount of the polymerizable monomer falls below the lower limit, the surface hardness of the polymer may become unsatisfactory and when the amount exceeds the upper limit, the polymer may become brittle.

A dipentaerythritol-based polymerizable monomer is advantageously contained in the component (A) of the present invention in an amount of preferably 1 to 80 parts by weight, more preferably 10 to 60 parts by weight, much more preferably 20 to 40 parts by weight (based on 100 parts by weight of the whole component (A)). When the amount of the dipentaerythritol-based polymerizable monomer falls below the lower limit, the surface hardness of the polymer

may become unsatisfactory and when the amount exceeds the upper limit, the polymer may become brittle.

The content of the component (A) is preferably 5 to 80 wt%, more preferably 10 to 65 wt%, much more preferably 15 to 65 wt% based on 100 wt% of the composition. When the content of the component (A) falls below the lower limit, adhesion performance may not become satisfactory and when the content exceeds the upper limit, the amount of a component preferred and required for obtaining the effect of the present invention such as an initiator becomes too small, thereby making it impossible to obtain satisfactory effects such as adhesion performance and cuttability disadvantageously.

In the dental curable composition of the present invention, examples of the polymerization initiator (B) include a polymerization initiator, a curing agent and an accelerator which can be used for dental materials and surgical materials. At least one of the following compounds is preferably contained, or they may be used in combination according to use conditions.

A polymerization initiator capable of radically polymerizing a polymerizable monomer may be used as the polymerization initiator.

That is, (B) an organic amine-based polymerization initiator is used in the present invention. The organic amine compound of the organic amine-based polymerization initiator can be suitably selected from aliphatic and aromatic organic amine compounds, and examples thereof include aromatic amines such as N,N-dimethylaniline, N,N-dimethyl-p-toluidine (DMPT), N,N-diethyl-p-toluidine, N,N-diethanol-p-toluidine (DEPT), N,N-dimethyl-p-tert-butylaniline, N,N-dimethylanisidine, N,N-dimethyl-p-chloroaniline, N,N-dimethylaminoethyl (meth)acrylate, N,N-diethylaminoethyl (meth)acrylate, N,N-dimethylaminobenzoic acid and alkyl esters thereof, N,N-diethylaminobenzoic acid (DEABA) and alkyl esters thereof and N,N-dimethylaminobenzaldehyde (DMABAd); and N-phenylglycine (NPG), N-tolylglycine (NTG), N-methyl-N-phenylglycine (NMePG), N-(2-carboxyphenyl)glycine (N2CPG), N-(2-hydroxyphenyl)glycine (N2HPG), N-(4-carboxyphenyl)glycine (N4CPG), N-(4-hydroxyphenyl)glycine (N4HPG), N-(4-methoxyphenyl)glycine (N4MPG), N-(methoxycarbonyl)-N-phenylglycine (NMCNPG) and N-(3-methacryloyloxy-2-hydroxypropyl)-N-phenylglycine (NPG-GMA) .

An aromatic amine having a carbonyl group represented by the following formula (I) or an aromatic amine represented by the following formula (II) is preferably used to ensure the polymerization curing of the dental curable composition of the present invention without using an organic peroxide and further improve the adhesion to the dentine of the dental curable composition.

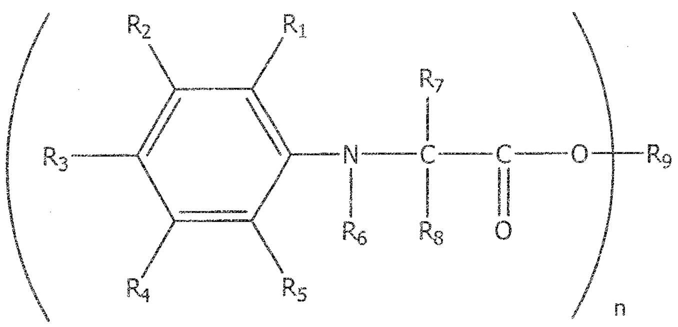

(I)

In the above formula (I), $R_1$, $R_2$, $R_3$, $R_4$ and $R_5$ are each independently a hydrogen atom, alkyl group, aryl group, aryloxy group, alkoxy group, nitro group, acyl group, acyloxy group, hydroxyl group or halogen atom, $R_6$ is a hydrogen atom, alkyl group or aryl group, $R_7$ and $R_8$ are each independently a hydrogen atom or alkyl group, $R_9$ is a hydrogen atom or metal atom, and n is 1 when $R_9$ is hydrogen and the same integer as the valence of a metal atom when $R_9$ is the metal atom.

The alkyl group represented by the substituents $R_1$, $R_2$, $R_3$, $R_4$ and $R_5$ in the above formula (I) is, for example, a linear, branched, monocyclic or condensed polycyclic alkyl group having 1 to 18 carbon atoms. Examples thereof include methyl group, ethyl group, propyl group, butyl group, pentyl group, hexyl group, heptyl group, octyl group, nonyl group, decyl group, dodecyl group, isopropyl group, isobutyl group, isopentyl group, sec-butyl group, tert-butyl group, sec-pentyl group, tert-pentyl group, tert-octyl group, neopentyl group, cyclopropyl group, cyclobutyl group, cyclopentyl group, cyclohexyl group, adamantyl group, norbornyl group, boronyl group and 4-decylcyclohexyl group. The alkyl group is not limited to these.

The aryl group represented by the substituents $R_1$, $R_2$, $R_3$, $R_4$ and $R_5$ in the above formula (I) is, for example, a monocyclic or condensed polycyclic aryl group having 4 to 18 carbon atoms which may contain a hetero atom. Examples thereof include phenyl group, 1-naphthyl group, 2-naphthyl group, 9-phenanthryl group, 1-pyrenyl group, 5-naphthacenyl group, 1-indenyl group, 2-azulenyl group, 1-acenaphthyl group, 2-furanyl group, 2-pyrrolyl group, 2-fluorenyl group, 2-furyl group, 2-thienyl group, 2-indolyl group, 3-indolyl group, 6-indolyl group, 2-benzofuryl group, 2-benzothienyl group,

4-quinolinyl group, 4-isoquinolyl group, 2-carbazolyl group, 3-carbazolyl group, 4-carbazolyl group, 9-acridinyl group, 3-phenothiazinyl group, 2-phenoxathinyl group, 3-phenoxazinyl group and 3-thianthrenyl group. The aryl group is not limited to these.

The aryloxy group represented by the substituents $R_1$, $R_2$, $R_3$, $R_4$ and $R_5$ in the above formula (I) is, for example, a monocyclic or condensed polycyclic aryloxy group having 4 to 18 carbon atoms which may contain a hetero atom. Examples thereof include phenoxy group, 1-naphthyloxy group, 2-naphthyloxy group, 9-anthryloxy group, 9-phenanthryloxy group, 1-pyrenyloxy group, 5-naphthacenyloxy group, 1-indenyloxy group, 2-azulenyloxy group, 1-acenaphthyloxy group, 9-fluorenyloxy group, 2-furanyloxy group, 2-thienyloxy group, 2-indolyloxy group, 3-indolyoxy group, 2-benzofuryloxy group, 2-benzothienyloxy group, 2-carbazolyloxy group, 3-carbazolyloxy group, 4-carbazolyloxy group and 9-acridinyloxy group. The aryloxy group is not limited to these.

The alkoxyl group represented by the substituents $R_1$, $R_2$, $R_3$, $R_4$ and $R_5$ in the above formula (I) is, for example, a linear, branched, monocyclic or condensed polycyclic alkoxyl group having 1 to 18 carbon atoms. Examples thereof include methoxy group, ethoxy group, propoxy group, butoxy group, pentyloxy group, hexyloxy group, heptyloxy group, octyloxy group, nonyloxy group, decyloxy group, dodecyloxy group, octadecyloxy group, isopropoxy group, isobutoxy group, isopentyloxy group, sec-butoxy group, tert-butoxy group, sec-pentyloxy group, tert-pentyloxy group, tert-octyloxy group, neopentyloxy group, cyclopropyloxy group, cyclobutyloxy group, cyclopentyloxy group, cyclohexyloxy group, adamantyloxy group, 2-tetrahydrofuranyloxy group and 2-tetrahydropyranyloxy group. The alkoxyl group is not limited to these.

The acyl group represented by the substituents $R_1$, $R_2$, $R_3$, $R_4$ and $R_5$ in the above formula (I) is, for example, a carbonyl group bonded to a hydrogen atom, a carbonyl group bonded to a linear, branched, monocyclic or condensed polycyclic aliphatic group having 1 to 18 carbon atoms, or a carbonyl group bonded to a monocyclic or condensed polycyclic aromatic group having 4 to 18 carbon atoms which may contain a hetero atom. It may have an unsaturated bond in the structure, and examples thereof include formyl group, acetyl group, propionyl group, butyryl group, isobutyryl group, valeryl group, isovaleryl group, pivaloyl group, lauroyl group, myristoyl group, palmitoyl group, stearoyl group, cyclopentylcarbonyl group, cyclohexylcarbonyl group, acryloyl group, methacryloyl group, crotonoyl group, isocrotonoyl group, oleoyl group, benzoyl group, 1-naphthoyl group, 2-naphthoyl group, cinnamoyl group, 3-furoyl group, 2-thenoyl group, nicotinoyl group and isonicotinoyl group. The acyl group is not limited to these.

The acyloxy group represented by the substituents $R_1$, $R_2$, $R_3$, $R_4$ and $R_5$ in the above formula (I) is, for example, a carbonyloxy group bonded to a hydrogen atom, a carbonyloxy group bonded to a linear, branched, monocyclic or condensed polycyclic aliphatic group having 1 to 18 carbon atoms, or a carbonyloxy group bonded to a monocyclic or condensed polycyclic aromatic group having 4 to 1.8 carbon atoms which may contain a hetero atom. Examples thereof include acetoxy group, propionyloxy group, butyryloxy group, isobutyryloxy group, valeryloxy group, isovaleryloxy group, pivaloyloxy group, lauroyloxy group, myristoyloxy group, palmitoyloxy group, stearoyloxy group, cyclopentylcarbonyloxy group, cyclohexylcarbonyloxy group, acryloyloxy group, methacryloyloxy group, crotonoyloxy group, isocrotonoyloxy group, oleoyloxy group, benzoyloxy group, 1-naphthoyloxy group, 2-naphthoyloxy group, cinnamoyloxy group, 3-furoyloxy group, 2-thenoyloxy group, nicotinoyloxy group, isonicotinoyloxy group, 9-anthroyloxy group and 5-naphthacenoyloxy group. The acyloxy group is not limited to these.

The alkyl group, aryl group, acyl group, alkoxyl group, aryloxy group and acyloxy group represented by the substituents $R_1$, $R_2$, $R_3$, $R_4$ and $R_5$ in the above formula (I) may be substituted by another substituent. Examples of the substituent include hydroxyl group, mercapto group, cyano group, nitro group, halogen atom, alkyl group, aryl group, acyl group, alkoxyl group, polyether group, aryloxy group, acyloxy group, alkylthio group and arylthio group.

The alkyl group represented by the substituent $R_6$ in the above formula (I) is, for example, a linear, branched, monocyclic or condensed polycyclic alkyl group having 1 to 12 carbon atoms. Examples thereof include methyl group, ethyl group, propyl group, butyl group, pentyl group, hexyl group, heptyl group, octyl group, nonyl group, decyl group, dodecyl group, isopropyl group, isobutyl group, isopentyl group, sec-butyl group, tert-butyl group, sec-pentyl group, tert-pentyl group, tert-octyl group, neopentyl group, cyclopropyl group, cyclobutyl group, cyclopentyl group, cyclohexyl group, adamantyl group and norbornyl group.

The aryl group represented by the substituent $R_6$ in the above formula (I) is, for example, a monocyclic or condensed polycyclic aryl group having 4 to 18 carbon atom which may contain a hetero atom. Examples thereof include phenyl group, 1-naphthyl group, 2-naphthyl group, 9-phenanthryl group, 1-pyrenyl group, 5-naphthacenyl group, 1-indenyl group, 2-azulenyl group, 1-acenaphthyl group, 2-furanyl group, 2-pyrrolyl group, 9-fluorenyl group, 2-furyl group, 2-thienyl group, 2-indolyl group, 3-indolyl group, 6-indolyl group, 2-benzofuryl group, 2-benzothienyl group, 4-quinolinyl group, 4-isoquinolyl group, 2-carbazolyl group, 3-carbazolyl group, 4-carbazolyl group, 9-acridinyl group, 3-phenothiazinyl group, 2-phenoxathinyl group, 3-phenoxazinyl group and 3-thianthrenyl group. The aryl group is not limited to these. The substituents represented by $R_1$, $R_2$, $R_3$, $R_4$ and $R_5$ may be bonded to an adjacent substituent to form a cyclic structure.

The alkyl group and aryl group represented by the substituent $R_6$ in the above formula (I) may be substituted by another substituent. Examples of the substituent include hydroxyl group, mercapto group, cyano group, nitro group,

halogen atom, alkyl group, aryl group, acyl group, alkoxyl group, aryloxy group, acyloxy group, alkylthio group and arylthio group.

The alkyl group represented by the substituents $R_7$ and $R_8$ in the above formula (I) is, for example, a linear, branched, monocyclic or condensed polycyclic alkyl group having 1 to 12 carbon atoms. Examples thereof include methyl group, ethyl group, propyl group, butyl group, pentyl group, hexyl group, heptyl group, octyl group, nonyl group, decyl group, dodecyl group, isopropyl group, isobutyl group, isopentyl group, sec-butyl group, tert-butyl group, sec-pentyl group, tert-pentyl group, tert-octyl group, neopentyl group, cyclopropyl group, cyclobutyl group, cyclopentyl group, cyclohexyl group, adamantyl group and norbornyl group.

The metal atom represented the substituent $R_9$ in the above formula (I) is preferably an alkali metal atom or alkali earth metal atom, more preferably potassium or sodium.

Examples of the organic amine compound represented by the above formula (I) include NPG, NTG, NMePG, N4HPG and NPG-GMA all of which are enumerated above. Out of these, NPG, N4HPG and NMePG are preferably used. Since salts (alkali metal salts and alkali earth metal salts) thereof have such high color stability that they hardly change their colors while they are kept at room temperature, potassium N-phenylglycine (NPG-K), sodium N-phenylglycine (NPG-Na), potassium N-methyl-N-phenylglycine (NMePG-K), sodium N-methyl-N-phenylglycine (NMePG-Na), potassium N-(4-hydroxyphenyl)glycine (N4HPG-K) and sodium N-(4-hydroxyphenyl)glycine (N4HPG-Na) are particularly preferably used.

When NPG is used out of the organic amine compounds used in the present invention, NPG containing a low content of impurities is preferably used. The impurities are generated when NPG forming no salt is left in the air at room temperature. Since these impurities may impede the effect of the present invention, it is preferred that the generation of the impurities should be suppressed or that the impurities should be removed accurately. That is, the total content of these impurities is preferably not more than 20 wt%, more preferably not more than 10 wt%, much more preferably not more than 5 wt% based on 100 wt% of NPG or a compound similar to NPG.

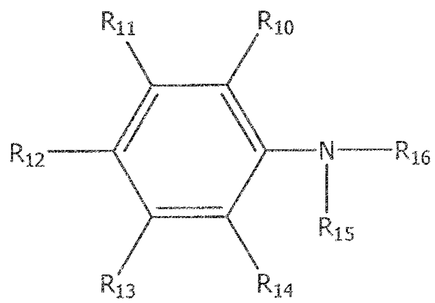

(II)

In the above formula (II), $R_{10}$, $R_{11}$, $R_{12}$, $R_{13}$ and $R_{14}$ are each independently a hydrogen atom, alkyl group, aryl group, aryloxy group, alkoxyl group, nitro group, acyl group, acyloxy group, hydroxyl group or halogen atom, and $R_{15}$ and $R_{16}$ are each independently a hydrogen atom, alkyl group, aryl group or substituted alkyl group containing a hetero atom.

Examples of the alkyl group and aryl group represented by the substituents $R_{10}$, $R_{11}$, $R_{12}$, $R_{13}$ and $R_{14}$ in the above formula (II) are the same as those enumerated for $R_1$ to $R_5$. The substituents represented by $R_{10}$, $R_{11}$, $R_{12}$, $R_{13}$ and $R_{14}$ may be bonded to an adjacent substituent to form a cyclic structure.

The alkyl group, aryl group, acyl group, alkoxyl group, aryloxy group and acyloxy group represented by the substituents $R_{10}$, $R_{11}$, $R_{12}$, $R_{13}$ and $R_{14}$ in the above formula (II) may be substituted by another substituent. Examples of the substituent include hydroxyl group, mercapto group, cyano group, nitro group, halogen atom, alkyl group, aryl group, acyl group, alkoxyl group, polyether group, aryloxy group, acyloxy group, alkylthio group and arylthio group.

The substituents $R_{15}$ and $R_{16}$ in the above formula (II) are each independently a hydrogen atom, alkyl group, aryl group, or substituted alkyl group containing a hetero atom. Examples thereof include methyl group, ethyl group, propyl group, butyl group, pentyl group, hexyl group, heptyl group and octyl group. Examples of the substituted alkyl group containing a hetero atom include halogen-substituted alkyl groups such as fluoromethyl group and 2-fluoroethyl group, and hydroxyl group-substituted alkyl groups such as 2-hydroxyethyl group and 3-hydroxypropyl group.

Examples of the organic amine compound represented by the above formula (II) include aliphatic alkylaminobenzoic acid and alkyl esters thereof typified by N,N-dimethylaminobenzoic acid and alkyl esters thereof, N,N-diethylaminobenzoic acid (DEABA) and alkyl esters thereof, N,N-dipropylaminobenzoic acid and alkyl esters thereof, N-isopropylaminobenzoic acid and alkyl esters thereof, and N-isopropyl-N-methylaminobenzoic acid and alkyl esters thereof; aliphatic alkyaminobenzaldehydes typified by DMABAd, N,N-diethylaminobenzaldehyde, N,N-dipropylaminobenzaldehyde and N-isopropyl-N-methylaminobenzaldehyde; and aliphatic alkylaminoacetylbenzenes typified by N,N-dimethylaminoacetylbenzene, N,N-diethylaminoacetylbenzene, N,N-dipropylaminoacetylbenzene, N-isopropylaminoacetylbenzene and N-isopropyl-N-methylaminoacetylbenzene, and aliphatic alkylaminoacylbenzenes. These organic amino compounds may be used alone or in combination.

The following initiators may be used in combination with the above amine compound as the component (B). The initiators include organic peroxides such as diacetyl peroxide, dipropyl peroxide, dibutyl peroxide, dilauryl peroxide, benzoyl peroxide (BPO), p,p'-dichlorobenzoyl peroxide, p,p'-dimethoxybenzoyl peroxide, p,p'-dimethylbenzoyl peroxide and p,p'-dinitrodibenzoyl peroxide, and inorganic peroxides such as ammonium persulfate, potassium persulfate, potassium chlorate, potassium bromate and potassium perphosphate.

Since an initiator which is a combination of an organic amine represented by the formula (I) or (II) and a peroxide has the following drawbacks, it is preferred to take them into consideration before use. That is, as for the organic amine represented by the formula (I) or (II), although a tertiary amine has higher radical generation ability than a secondary amine, it hardly dissolves in water and an organic peroxide also hardly dissolves in water. In addition, a radical generated from an organic peroxide is readily deactivated by water. Therefore, since the initiator is apt to stay in the polymerizable composition rather than on the surface of the dentine rich in water to polymerize the polymerizable monomer, polymerization on the surface of the dentine hardly proceeds, whereby micro-leakage may occur. Since an inorganic peroxide is a salt, in general, it has high water solubility and high oxidation power, whereby the discoloration of the amine may become marked, thereby staining a cured product.

To solve these problems, the above peroxide is used in an amount of preferably not more than 70 parts by weight, more preferably not more than 3.5 parts by weight, much more preferably not more than 0.05 part by weight based on 100 parts by weight of the compound represented by the formula (I) or (II). Alternatively, the above peroxide is used in an amount of preferably not more than 2 parts by weight, more preferably not more than 0.1 part by weight, much more preferably not more than 0.005 part by weight based on 100 parts by weight of the total of the components (A) and (B) .

Further, an organic boron compound or a composition containing the same may be used in combination with the above amine compound which is the component (B). Examples of the organic boron compound include trialkylborons such as triethylboron, tripropylboron, triisopropylboron, tributylboron, tri-sec-butylboron, triisobutylboron, tripentylboron, trihexylboron, trioctylboron, tridecylboron, tridodecylboron, tricyclopentylboron and tricyclohexylboron; alkoxyalkylborons such as butoxydibutylboron; and dialkylboranes such as butyldicyclohexylborane, diisoamylborane and 9-borabicyclo[3,3,1]nonane. The above compounds may be partially oxidized. Further, these compounds may be used in combination. Out of these, tributylboron or partially oxidized tributylboron, for example, an adduct of 1 mol of tributylboron with 0.3 to 0.9 mol of $O_2$ is preferably used. A composition comprising an organic boron compound and an aprotic solvent and/or a liquid or solid organic oligomer or polymer which is inactive to the organic boron compound may also be used.

A composition comprising an organic boron compound and an aprotic solvent and/or a liquid or solid organic oligomer or polymer which is inactive to an organic boron compound may also be used.

Arylboron derivatives may be used as the organic boron compound besides the above alkylboron derivatives. Borate compounds having 1 to 4 boron-aryl bonds in one molecule may be used as the arylboron derivatives. Borate compounds having 3 boron-aryl bonds include sodium, lithium, potassium, magnesium, tetrabutylammonium, tetramethylammonium, tetraethylammonium, tributylammonium triethanolammonium, methylpyridinium, ethylpyridinium, butylpyridinium, methylquinolinium, ethylquinolinium and butylquinolinium salts of monoalkyl triphenylboron, monoalkyl tris(p-chlorophenyl)boron, monoalkyl tris(p-fluorophenyl)boron, monoalkyl tris(3,5-bistrifluoromethyl)phenylboron, monoalkyl tris[3,5-bis(1,1,1,3,3,3-hexafluoro-2-methoxy-2-propyl) phenyl]boron, monoalkyl tris(p-nitrophenyl)boron, monoalkyl tris(m-nitrophenyl)boron, monoalkyl tris(p-butylphenyl)boron, monoalkyl tris(m-butylphenyl)boron, monoalkyl tris(p-butyloxyphenyl)boron, monoalkyl tris(m-butyloxyphenyl)boron, monoalkyl tris(p-octyloxyphenyl)boron and monoalkyl tris(m-octyloxyphenyl)boron (the alkyl is any one of n-butyl, n-octyl and n-dodecyl in these compounds).

Borate compounds having 4 boron-aryl bonds in one molecule include sodium, lithium, potassium, magnesium, tetrabutylammonium, tetramethylammonium, tetraethylammonium, tributylammonium triethanolammonium, methylpyridinium, ethylpyridinium, butylpyridinium, methylquinolinium, ethylquinolinium and butylquinolinium salts of tetraphenylboron, tetrakis(p-chlorophenyl)boron, tetrakis(p-fluorophenyl)boron, tetrakis(3,5-bistrifluoromethyl)phenylboron, tetrakis[3,5-bis(1,1,1,3,3,3-hexafluoro-2-methoxy-2-propyl)phenyl]boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron and tetrakis(m-octyloxyphenyl)boron (the alkyl is any one of n-butyl, n--octyl and n-dodecyl in these compounds).

These arylboron derivatives are compounds enumerated in JP-A 2002-187907 and JP-A 2003-96122.

The organic boron compound is used in an amount of preferably not more than 2,000 parts by weight, more preferably not more than 500 parts by weight, much more preferably not more than 10 parts by weight based on 100 parts by weight of the compound represented by the formula (I) or (II). When the amount of the organic boron compound exceeds the above range, a problem that the polymerization rate becomes too high occurs disadvantageously.

The curable composition of the present invention can also be polymerized by exposure to ultraviolet light or visible light. A photopolymerization initiator used for the composition is excited by light by itself or in the presence of another compound to cure the dental curable composition of the present invention. The photopolymerization initiator is

selected from an α-ketocarbonyl compound and an acylphosphine oxide compound.

The α-ketocarbonyl is selected from an α-diketone, α-ketoaldehyde, α-ketocarboxylic acid and α-ketocarboxylic acid ester. Specific examples thereof include α-diketones such as diacetyl, 2,3-pentadione, 2,3-hexadione, benzyl, 4,4'-dimethoxybenzyl, 4,4'-diethoxybenzyl, 4,4'-oxybenzyl, 4,4'-dichlorobenzyl, 4-nitrobenzyl, α-naphthyl, β-naphthyl, camphorquinone, camphorquinonesulfonic acid, camphorquinonecarboxylic acid and 1,2-cyclohexanedione; α-ketoaldehydes such as methylglyoxal and phenylglyoxal; and pyruvic acid, benzoylformic acid, phenylpyruvic acid, methyl pyruvate, ethylbenzoyl formate, methyl phenylpyruvate and butyl phenylpyruvate. Out of these α-ketocarbonyl compounds, a-diketones are preferably used from a view point of stability. Out of the α-diketones, diacetyl, benzyl and camphorquinone are preferred.

Examples of the acylphosphine oxide compound include benzoyl dimethoxyphosphine oxide, benzoyl ethoxyphenylphosphine oxide, benzoyl diphenylphosphine oxide, 2-methylbenzoyl diphenylphosphine oxide and 2, 4, 6-trimethylbenzoyl phenylphosphine oxide. These α-ketocarbonyl compounds and the acylphosphine oxide compounds may be used alone or in combination. When they are used in combination, a combination of camphorquinone and diphenyltrimethylbenzoylphosphine oxide is particularly preferably used.

The photopolymerization initiator is used in an amount of preferably not more than 200 parts by weight, more preferably not more than 10 parts by weight, much more preferably not more than 0.1 part by weight based on 100 parts by weight of the compound represented by the formula (I) or (II). Or, the photopolymerization initiator is used in an amount of preferably 0.0001 to 5 parts by weight, more preferably 0.0005 to 0.1 part by weight, much more preferably 0.001 to 0.01 part by weight based on 100 parts by weight of the total of the components (A) and (B). When the amount of the photopolymerization initiator falls below the lower limit, curing by exposure becomes difficult due to unsatisfactory photosensitization ability. When the amount exceeds the upper limit, polymerization in the inside of the polymerizable composition proceeds excessively at the time of exposure, thereby causing interfacial delamination between the cured product and the dentine or micro-leakage disadvantageously.

(C) A soft resin material having a durometer A hardness of not more than 90 or a durometer D hardness of not more than 60 is preferably used in the dental curable composition of the present invention. More preferably, the soft resin material has a durometer A hardness of not more than 70 or a durometer D hardness of not more than 50. Much more preferably, the soft resin material has a durometer A hardness of not more than 60 or a durometer D hardness of not more than 30. When the durometer A hardness is higher than 90 or the durometer D hardness is higher than 60, it may be difficult to remove a cured product of the dental curable composition at the time of retreating a root canal disadvantageously. There are three different types of durometers based on JIS K6253 which are durometers for measuring middle hardness (type A durometer), high hardness (type D durometer) and low hardness (type E durometer). Numerical values obtained by these different types of hardness meters are shown as durometer A hardness, durometer D hardness and durometer E hardness, respectively. In general, it is recommended to use a type E durometer when the hardness value measured by a type A durometer based on JIS K6253 is less than 20 and a type D durometer when the hardness value is more than 90.

Therefore, as for the above upper limit value, at least the durometer A hardness preferably satisfies the above numerical range. Although the lower limit value is not particularly limited, in consideration of the above recommendation (the durometer E hardness parameter is used when the durometer A hardness is less than 20), the durometer E hardness should be used according to the situation. Preferably, the durometer A hardness is not less than 20 or the durometer E hardness is not less than 1, more preferably not less than 5, much more preferably not more than 10. When the numerical value falls below the above lower limit and the soft resin material is in a pellet form and not a powdery form, grinding work for reducing the particle size to a desired size becomes difficult disadvantageously. The soft resin material is preferably powdery and, when it is powdery, its particle size is not particularly limited. However, when the dispersibility into the component (A) and packing property of the soft resin material are taken into consideration, its median particle diameter (a value obtained by isometrically changing a non-sphere into a sphere, or a measurement value obtained by the laser diffraction/scattering particle size distribution measuring instrument of HORIBA, Ltd.) which means a 50 % particle size of an integral particle size distribution curve is preferably 0.01 to 500 μm, more preferably 0.1 to 100 pm, much more preferably 1 to 50 pm, particularly preferably 2 to 20 pm. When applied to a tooth, if the soft resin material is existent as a domain of the above size in a cured product, the cured product is easily removed advantageously. That is, the soft resin material (C) is a resin which is not substantially dissolved by the component (A), more preferably a resin which has low compatibility with the component (A), a polymer thereof and a component (J) which will be described hereinafter. The boundary of the domain may be made unclear microscopically by a solvent or a monomer having compatibility with the soft resin material (C). When the soft resin material (C) has extremely high compatibility with the cured product and is completely mixed with the cured product, the deterioration of adhesion at the interface with the dentine may occur disadvantageously.

The soft resin material (C) should be existent in a particulate form as a domain as described above when the component (A) is polymerized to be cured. The form of the soft resin material (C) in the composition may be arbitrary before use. For example, the soft resin material (C) may be kept as a powdery composition or an agglomerate independently from a liquid monomer composition. To save the trouble of dispersion, the soft resin material may be existent in a particulate form in a liquid or paste-like composition containing a monomer.

The soft resin material can be selected from so-called "soft resins" and can be obtained by selecting the type of molecules constituting the resin and adjusting the molecular weight, dispersion and copolymerization ratio of the resin and the types and amounts of materials to be mixed together. Examples of the soft resin material include gutta-percha, polyethylene, polypropylene, ethylene propylene copolymer, ethylene propylene terpolymer, silicone polymer, polyisoprene, ethylene vinyl acetate copolymer, and ethylene- (meth) acrylate copolymers such as ethylene-methyl (meth)acrylate copolymer, ethylene-ethyl (meth)acrylate copolymer and ethylene-butyl (meth)acrylate copolymer. It is preferred that at least one selected from these should be contained.

The above gutta-percha refers to a material which comprises polyisoprene as natural rubber or a rubber-like material (natural or synthetic polymer) comprising polyisoprene as the main component and additives, an inorganic compound X-ray contrast agent (such as zinc oxide, barium sulfate or heavy metal salt) or an organic compound such as wax. The gutta-percha is known per se and can be acquired in various forms. In the present invention, the terms "gutta-percha point", "gutta-percha corn" and "gutta-percha chip" are used to express a sewing needle-like cured product containing gutta-percha, a small candle-like cylindrical cured product and a granular cured product larger than a powder, respectively. Polyisoprene is available as a synthetic polymer in cis-polyisoprene, trans-polyisoprene and atactic polyisoprene which is a mixture of these polyisoprenes, out of which trans-polyisoprene is preferably used. The above natural and synthetic polymers may be used alone or in combination in the component (A).

As for the composition of the dental curable composition of the present invention, when the amount based on 100 parts by weight of the component (A) of the component (A') contained in the component (A) is represented by [a'] and the amounts based on 100 parts by weight of the total of the components (A) and (B) of the components (A), (B) and (C) are represented by (a), (b) and (c), respectively, $70 \leqq (a) \leqq 99.99$, $0.01 \leqq (b) \leqq 30$, $1 \leqq [a']/5 + (c)/1$, $0 \leqq [a'] \leqq 95$, and $0 \leqq (c) \leqq 250$.

More preferably, the amount of the component (A) is 80 to 99.95 parts by weight, and the amount of the component (B) is 0.05 to 20 parts by weight. Much more preferably, the amount of the component (A) is 90 to 99.9 parts by weight, and the amount of the component (B) is 0.1 to 10 parts by weight. The total of the components (A) and (B) is 100 parts by weight.

When the amount of the component (A) falls below the lower limit or the amount of the component (B) exceeds the upper limit, the time color change of the cured product due to the component (B) in the oral cavity increases, and when the amount of the component (A) exceeds the upper limit or the amount of the component (B) falls below the lower limit, polymerization becomes incomplete disadvantageously.

When the amount based on 100 parts by weight of the component (A) of the component (A') contained in the component (A) is represented by [a'] and the amount based on 100 parts by weight of the total of the components (A) and (B) of the component (C) is represented by (c), $1 \leqq [a']/5 + (c)/1$, $0 \leqq [a'] \leqq 95$, and $0 \leqq (c) \leqq 250$.

A detailed description is given of the relational expression between the component (A') and the component (C).

As for the definition of [a'], since the component (A') belongs to the subordinate concept of the component (A), $[a'] = W_{(A')}/W_{(A)}=W_{(A')}/(W_{(A')}+W^{-}_{(A')})$ ($W_{(A')}$ = weight of component (A'), $W_{(A)}$ = weight of component (A), $W^{-}_{(A')}$ = weight of component (A) except for component (A')).

The critical significances of the lower limits of the amounts of the component (A') and the component (C) are connected with the effect of removability and work independently in function and mechanism. In other words, they have a linearly combined relationship mathematically. Therefore, if the amounts of these components fall below the lower limits when they are used alone, it is possible that they are compensated for each other to produce the effect of the present invention.

First, the conditions concerning the lower limit values when they are used alone are defined as follows. $A'_1 \leqq A'_i$ ($A'_i$ is the amount (parts by weight) of the component (A') and $A'_1$ is the lower limit value of the amount of the component (A'))

$C_1 \leqq C_i$ ($C_i$ is the amount (parts by weight) of the component (C) and $C_1$ is the lower limit value of the amount of the component (C))

Since the lower limit values of the amounts of these components differ from each other, both sides are divided by the lower limit values to be normalized.

$$1 \leqq A'_i / A'_1$$

$$1 \leqq C_i / C_1$$

That is, when all of the above values exceed 1, the effect of the present invention is obtained. However, even when both of them are less than 1, if the total of them is not less than 1, it is considered that the effect of the present invention is obtained. Therefore, when the both components are coexistent and the following condition is satisfied, the effect of the present invention should be guaranteed.

$$1 \leqq A'_i / A'_1 + C_i / C_1$$

It is needless to say that the above relational expression becomes an expression showing the lower limit value of the amount of one component when the amount of the other component is 0.

Therefore, as for the lower limit values of the amounts of the component (A') and the component (C) when they are used alone, the lower limit of the amount of the component (A') is 5 parts by weight (based on 100 parts by weight of the component (A) containing the component (A')) and the lower limit of the amount of the component (C) is 1 part by weight (based on 100 parts by weight of the total of the components (A) and (B)). Therefore, by inserting these values into $A'_1$ and $C_1$, the above relational expression is obtained.

When they are used alone, the lower limit of the amount of the component (A') is preferably 9 parts by weight, more preferably 15 parts by weight, and the lower limit of the amount of the component (C) is preferably 30 parts by weight, more preferably 50 parts by weight. It is needless to say that, by inserting these values into $A'_1$ and $C_1$, a preferred relational expression is obtained.

As for the upper limit values, when the amount (parts by weight) based on 100 parts by weight of the component (A) of the component (A') contained in the component (A) is represented by [a'] and the amount (parts by weight) based on 100 parts by weight of the total of the components (A) and (B) of the component (C) is represented by (c), 0 ≦ [a'] ≦ 95, preferably 0 ≦ [a'] ≦ 70, more preferably 0 ≦ [a'] ≦ 50, and 0 ≦ (c) ≦ 250, preferably 0 ≦ (c) ≦ 200, more preferably 0 ≦ (c) ≦ 150.

When the amount of the component (A') exceeds the above upper limit, the amount of the main chain of the polymerization structure in the formed polymer relatively decreases with the result that the polymer may become brittle and unsatisfactory in terms of strength. Particularly when the above component (A') comprises a long-chain oxyalkylene recurring unit, its compatibility with a hydrophobic monomer when it is polymerized may lower or the water resistance of the polymerized cured product may degrade disadvantageously. When the amount of the component (C) exceeds the above upper limit, the amount of the polymerizable monomer which can infiltrate into the dentine decreases, whereby a high-quality layer structure in which different components are interpenetrated with each other three-dimensionally is not formed, thereby degrading marginal sealability disadvantageously. Since the reason that it is not preferred that the amount exceeds the upper limit differs according to each component, the above components are independent without being interfered with each other.

The amount of the polymerizable monomer having an acid group contained in the component (A) is preferably 1 to 40 parts by weight, more preferably 3 to 30 parts by weight, much more preferably 5 to 25 parts by weight based on 100 parts by weight of the component (A) . When the amount of the polymerizable monomer falls below the lower limit, the effect of promoting the infiltration into the dentine of the component (A) having no acid group is reduced or the decalcification of the dentine becomes unsatisfactory. When the amount of the polymerizable monomer exceeds the upper limit, the decalcification of the dentine becomes excessive disadvantageously as well.

The amount of the component (A') having a chain length of 32 or less atoms or 9 or less recurring units derived from ethylene glycol is preferably 10 to 97 parts by weight, more preferably 25 to 80 parts by weight, much more preferably 35 to 60 parts by weight.

The amount of the polyfunctional polymerizable monomer contained in the component (A) is preferably 5 to 80 parts by weight, more preferably 20 to 70 parts by weight, much more preferably 30 to 60 parts by weight based on 100 parts by weight of the component (A). When the amount of the polyfunctional polymerizable monomer falls below the lower limit, polymerization becomes incomplete and when the amount exceeds the upper limit, removal becomes difficult disadvantageously.

The weight ratio of {polyfunctional polymerizable monomer as component (A')}/{polyfunctional polymerizable monomer as component (A') + polyfunctional polymerizable monomer which is not component (A')} in the component (A) is preferably 0 to 100 %, more preferably 30 to 100 %, much more preferably 60 to 100 %.

Alternatively, the amount of the polyfunctional polymerizable monomer which is the component (A') is preferably 1 to 70 parts by weight, more preferably 10 to 50 parts by weight, much more preferably 20 to 40 parts by weight based on 100 parts by weight of the component (A). The amount of the polyfunctional polymerizable monomer which is not the component (A') is preferably 1 to 50 parts by weight, more preferably 5 to 30 parts by weight, much more preferably 10 to 20 parts by weight based on 100 parts by weight of the component (A). When the amount of the polyfunctional polymerizable monomer is equal to or larger than the above lower limit, adhesion to the dentine is stabilized and interfacial

adhesion is improved even with a small amount of a monomer having a water-soluble group advantageously. However, when the amount exceeds the upper limit, removal becomes difficult disadvantageously.

When the amount of the polyfunctional polymerizable monomer which is the component (A') falls below the above lower limit, cuttability degrades and when the amount exceeds the upper limit, the amount of the main chain of the polymerization structure in the formed polymer decreases relatively with the result that the obtained polymer may become brittle and unsatisfactory in terms of strength. Particularly when the above component (A') comprises a long-chain oxyalkylene recurring unit, its compatibility with a hydrophobic monomer may degrade when it is polymerized, or the water resistance of the polymerized cured product may lower disadvantageously. When the amount of the polyfunctional polymerizable monomer which is not the component (A') falls below the above lower limit, it is difficult for the polyfunctional polymerizable monomer to infiltrate into the dentine to be crosslinked, whereby the curing rate may become low when a polymerizable composition is prepared, or the viscosity may become too low. When the amount exceeds the upper limit, micro-leakage may occur due to polymerization shrinkage, the curing rate may become too high when a polymerizable composition is prepared, or the viscosity may become too high disadvantageously.

As for the component (A), monomers can be classified into 16 groups according to the length of the molecular chain, the existence of an acid group, whether they are monofunctional or polyfunctional and the existence of a hydrophilic group such as an alcoholic hydroxyl group (aromatic hydroxyl group and carboxyl group are hydrophobic). Both monofunctional and polyfunctional long-chain monomers are important, and whether long-chain monomers have an acid group or not and a hydrophilic group or not is not so important. Whether short-chain monomers have an acid group or not is important and then whether they are monofunctional or polyfunctional is important. Therefore, the components (A) can be roughly classified into the following six groups.

(1) Long-chain polyfunctional monomers (may have an acid group and/or a hydrophilic group)
(2) Long-chain monofunctional monomers (may have an acid group and/or a hydrophilic group)
(3) Short-chain monomers having an acid group (may be polyfunctional and/or may have a hydrophilic group)
(4) Short-chain polyfunctional monomers having no acid group (may have a hydrophilic group)
(5) Monofunctional short-chain monomers having no acid group and no hydrophilic group
(6) Short-chain monofunctional monomers having a hydrophilic group and no acid group

The component (1) is contained in an amount of preferably 1 to 70 parts by weight, more preferably 10 to 50 parts by weight, much more preferably 20 to 40 parts by weight based on 100 parts by weight of the component (A). When the amount of the component (1) falls below the above lower limit, removal becomes difficult and when the amount exceeds the upper limit, the obtained polymer becomes too brittle disadvantageously.

The component (2) is contained in an amount of preferably 0 to 70 parts by weight, more preferably 5 to 50 parts by weight, much more preferably 10 to 30 parts by weight based on 100 parts by weight of the component (A). When the amount of the component (2) is equal to or larger than the above lower limit, removal becomes easy advantageously but when the amount exceeds the upper limit, polymerizability degrades disadvantageously.

The component (3) is contained in an amount of preferably 1 to 40 parts by weight, more preferably 7 to 30 parts by weight, much more preferably 10 to 25 parts by weight based on 100 parts by weight of the component (A). When the amount of the component (3) falls below the above lower limit, the decalcification of the dentine becomes unsatisfactory and when the amount exceeds the upper limit, the dentine becomes overdecalcified disadvantageously.

The component (4) is contained in an amount of preferably 0 to 60 parts by weight, more preferably 0 to 40 parts by weight, much more preferably 0 to 20 parts by weight based on 100 parts by weight of the component (A). When the amount of the component (4) is equal to or larger than the above lower limit, adhesion to the dentine is stabilized and interfacial adhesion is improved even with a small amount of a monomer having a water-soluble group advantageously. However, when the amount exceeds the upper limit, removal becomes difficult disadvantageously.

The component (5) is contained in an amount of preferably 0 to 30 parts by weight, more preferably 0 to 20 parts by weight, much more preferably 0 to 10 parts by weight based on 100 parts by weight of the component (A). When the amount of the component (5) is equal to or larger than the above lower limit, the water resistance of a cured product improves advantageously and when the amount exceeds the upper limit, its compatibility with the component (A') degrades disadvantageously.

The component (6) is contained in an amount of preferably 5 to 70 parts by weight, more preferably 20 to 60 parts by weight, much more preferably 30 to 50 parts by weight based on 100 parts by weight of the component (A). When the amount of the component (6) falls below the above lower limit, adhesion to the dentine becomes unstable and when the amount exceeds the upper limit, a volume change (swelling) by the water absorption of a cured product becomes marked disadvantageously.

The dental curable composition may comprise (X) a component which does not belong to all of the above components (A) to (C), and the amount of the component (X) is preferably not more than 400 parts by weight based on 100 parts by weight of the total of the components (A) and (B) which are not overlapped with the component (X). The

component (X) includes components (D) to (J) which will be described below. When the component (X) is compatible with a mixture of the components (A) and (B), the amount of the component (X) is preferably not more than 100 parts by weight.

The dental curable composition according to a second aspect of the present invention comprises (D) a transition metal compound in addition to the above components (A), (B) and (C) and/or the following component (G). Examples of the transition metal compound (D) used herein include manganese fluoride, iron chloride, cobalt fluoride and copper chloride.

More specifically, the transition metal of the transition metal compound may belong to any one of first transition series, second transition series, third transition series, lanthanide series and actinide series and is not particularly limited. Out of the first transition series, manganese, iron, cobalt and copper transition metal compounds are preferred, and manganese, iron and copper transition metal compounds are more preferred. Besides the first transition series, a cerium transition metal compound is particularly preferably used.

The oxidation number of the transition metal contained in the transition metal compound used in the present invention is not particularly limited. The properties of the transition metal compound differ according to the oxidation number of the transition metal. For example, although iron compounds having a valence of 2 or less and iron compounds having a valence of 3 or more have excellent polymerization activity, the iron compounds having a valence of 2 or less have an advantage that they are rarely stained purplish red and a disadvantage that they have low stability to oxygen in the air. Meanwhile, trivalent iron has an advantage that it is stable to oxygen in the air and a disadvantage that it is greatly stained purplish red. Since the properties of the transition metal compound differ according to the oxidation number of the contained transition metal as described above, the oxidation number of the transition metal may be selected in consideration of the difference in properties and the use purpose of the present invention or the oxidation-reduction potential of the transition element to control the polymerization rate. As for the oxidation-reduction potential of each transition element, scandium has an oxidation-reduction potential of -2.08 E° /V, titanium has an oxidation-reduction potential of -1.63 E° /V or -0.37 E° /V, vanadium has an oxidation-reduction potential of -1.13 E° /V or -0.26 E° /V, chromium has an oxidation-reduction potential of -0.79 E° /V or -0.42 E° /V, manganese has an oxidation-reduction potential of -1.18 E° /V or -1.51 E° /V, iron has an oxidation-reduction potential of -0.44 E° /V or 0.77 E° /V, cobalt has an oxidation-reduction potential of -0.29 E° /V or 1. 92 E° /V, nickel has an oxidation-reduction potential of -0.23 E° /V, copper has an oxidation-reduction potential of 0.15 E° /V, 0.34 E° /V or 0.52 E° /V and zinc has an oxidation-reduction potential of -0.76 E° /V. E° is a standard electrode potential.

Examples of the transition metal compound include salts with an inorganic acid or an organic acid, oxides and alloys of a transition metal. Examples of the inorganic acid include hydrochloric acid, hydrofluoric acid, sulfuric acid, nitric acid, phosphoric acid and carbonic acid. Out of these, hydrochloric acid and sulfuric acid are preferred. Examples of the organic acid include carboxylic acid and sulfinic acid having an organic group, phenol, enol, thiophenol, imide, oxime, aromatic sulfonamide, and primary and secondary nitro compounds. Out of these, carboxylic acid and enol are preferred.

Specific examples of the transition metal compound containing iron having a valence of 3 or more as the transition metal include iron chloride (III), iron fluoride (III), iron sulfate (III), iron nitrate (III), iron phosphate (III) and hydrates thereof; iron formate (III), iron acetate (III), iron propionate (III), iron acrylate (III), iron oxalate (III), iron citrate (III), iron gluconate (IIII), iron 2-ethylhexanoate (III), iron lactate (III) and iron naphthenate (III) of a monocarboxylic acid, iron fumarate (III) and iron maleate (III) of a dicarboxylic acid, iron polyacrylate (III) of a polycarboxylic acid, iron L-ascorbate of an enol, and hydrates thereof; and iron oxide (III), ferric (IV) acid salts and ferric (V) acid salts. Specific examples of the transition metal compound containing iron having a valence of 2 or less as the transition metal include iron chloride (II), iron fluoride (II), iron sulfate (II), iron nitrate (II), iron 2-ethylhexanoate (II), iron lactate (II), iron naphthenate (II), iron fumarate (II), iron maleate (II) of a dicarboxylic acid, iron acrylate (II) of a polycarboxylic acid, iron L-ascorbate (II) of an enol and hydrates thereof; iron oxide (II); and iron alloys.

The transition metal compound may be dispersed into the polymerizable monomer as it is, or may be dissolved or dispersed in a suitable medium and then added to the polymerizable monomer. Water, a hydrophilic solvent such as alcohol, or an aprotic solvent such as acetone or dimethyl sulfoxide is preferred as the medium for dissolving the transition metal compound.

The dental curable composition according to the second aspect of the present invention comprises the component (D) in an amount of preferably 0.001 to 10 parts by weight, more preferably 0.003 to 5 parts by weight, much more preferably 0.005 to 0.1 part by weight based on 100 parts by weight of the total of the components (A) and (B). It should be understood that the relative ratio of the components (A), (B) and (C) is the same as in the first dental curable composition.

The dental curable composition according to a third aspect of the present invention further comprises (E) a sulfur-containing reducing compound in addition to the above components (A), (B) and (C) and/or the following component (G) and the optional component (D) . In the dental curable composition of the present invention, the sulfur-containing reducing compound (E) is, for example, an organic sulfur-containing compound or an inorganic sulfur-containing compound. Examples of the organic sulfur-containing compound include aromatic sulfinic acids and salts thereof such as benzenesulfinic acid, o-toluenesulfinic acid, p-toluenesulfinic acid, ethylbenzenesulfinic acid, decylbenzenesulfinic acid, dodecylbenzenesulfinic acid, chlorobenzenesulfinic acid and naphthalinesulfinic acid, and aromatic sulfonic acids and salts thereof such as benzenesulfonic acid, o-toluenesulfonic acid, p-toluenesulfonic acid, ethylbenzenesulfonic acid, decylbenzenesulfonic acid, dodecylbenzenesulfonic acid, chlorobenzenesulfonic acid and naphthalinesulfonic acid. Out of these, p-toluenesulfinates are preferably used, and sodium p-toluenesulfinate is particularly preferably used.

Examples of the inorganic sulfur-containing compound include sulfurous acid, bisulfurous acid, metasulfurous acid, metabisulfurous acid, pyrosulfurous acid, thiosulfuric acid, 1-thionous2-thionic acid, 1,2-thionic acid, hyposulfurous acid, hydrosulfurous acid and salts thereof. Out of these, sulfites are preferably used, and sodium sulfite, potassium sulfite, sodium hydrogen sulfite and potassium hydrogen sulfite are particularly preferred. These organic and inorganic sulfur-containing compounds may be used alone or in combination.

The dental curable composition according to the third aspect of the present invention comprises the component (E) in an amount of preferably 0.01 to 10 parts by weight, more preferably 0.1 to 5 parts by weight, much more preferably 0.3 to 3 parts by weight based on 100 parts by weight of the total of the components (A) and (B). It should be understood that the relative ratio of the components (A), (B) and (C) is the same as in the first dental curable composition.

The dental curable composition according to a fourth aspect of the present invention further comprises (F) hydroxylic acid in addition to the above components (A), (B) and (C) and/or the following composition (G) and the optional components (D) and (E). The hydroxylic acid (F) used herein is not particularly limited if it is a compound containing a hydroxyl group and a carboxyl group. However, a compound having a hydroxyl group at the $\alpha$-position of a carboxylic acid group is preferably used. Examples of the compound include malic acid, tartaric acid, citric acid, glycolic acid, gluconic acid, $\alpha$-oxyisoacetic acid, 2-hydroxypropionic acid, 3-hydroxybutanoic acid, 4-hydroxybutanoic acid and dimethylolpropionic acid. These hydroxylic acid compounds may be used alone or in combination of two or more without problems.

The dental curable composition according to the fourth aspect of the present invention comprises the component (F) in an amount of preferably 0.01 to 10 parts by weight, more preferably 0.05 to 5 parts by weight, much more preferably 0.1 to 1 part by weight based on 100 parts by weight of the total of the components (A) and (B). It should be understood that the relative ratio of the components (A), (B), (C) and (D) in the composition is the same as in the first and second dental curable compositions.

As a matter of course, a solvent which is not covalently bonded to a monomer or a cured product by copolymerization should be selected in principle as the solvent of the above component (G). The solvent (G) used herein is preferably a solvent having compatibility with the long-chain polymerizable monomer (A'). Compatibility is not particularly limited but preferably such that not less than 3 parts by weight of the long-chain polymerizable monomer (A') can be uniformly dissolved in 100 parts by weight of the above solvent. However, there will be no problem if a homogeneous composition is obtained eventually even when a homogeneous solution is not produced by mixing together the solvent and each of the other components. It is recommended to use, for example, a polymerizable monomer having suitable compatibility (such as 2-hydroxyethyl (meth)acrylate) in combination.

Particularly when the long-chain polymerizable monomer (A') has a recurring unit derived from an oxyalkylene structure, especially ethylene glycol, the solvent is water alone or a mixed solvent of water and an organic solvent miscible with water. Examples of the water which can be used herein include distilled water and ion exchange water. A physiological saline solution may also be used as the aqueous solvent. Out of these, distilled water and ion exchange water are preferably used. Examples of the above organic solvent miscible with water include alcohols such as methanol, ethanol and propanol, ketones such as acetone and methyl ethyl ketone, ethers such as tetrahydrofuran, amides such as N,N-dimethylforamide, and aprotic solvents such as dimethyl sulfoxide. In consideration of damage and stimulus to the dental pulp, out of these organic solvents, ethanol and acetone are particularly preferably used.

The dental curable composition containing the component (G) of the present invention comprises the composition in an amount of 0.1 to 300 parts by weight, more preferably 1 to 150 parts by weight, more preferably 3 to 90 parts by weight, particular preferably 4 to 50 parts by weight, most preferably 10 to 30 parts by weight based on 100 parts by weight of the total of the components (A) and (B). When the amount of the component (G) falls below the above lower limit, cuttability may degrade and when the amount exceeds the upper limit, compatibility with the monomer may deteriorate disadvantageously.

It should be understood that the relative ratio of the components (A), (B), (C), (D) and (G) in the composition is the same as in the first, second and third dental curable compositions. When the ratio of the component (G) is high, primer properties tend to appear strong, and the composition can be actually used as a primer in this case.

It is particularly notable that when the long-chain polymerizable monomer having a chain length of 19 or more atoms (A') which is a polyalkylene-based, especially a polyglycol-based monomer has more than 9 polyalkylene units, its compatibility with water as the component (G) becomes markedly high. In addition, it is observed that cuttability is greatly improved by the component (A') in the presence of water. Then, the component (G), especially water is contained in an amount of preferably 1 to 300 wt%, more preferably 10 to 150 wt%, much more preferably 30 to 100 wt% based

on 100 wt% of the component (A').

When the amount of the component (G) falls below the above lower limit, cuttability degrade and when the amount exceeds the upper limit, compatibility with the monomer may deteriorate disadvantageously.

The dental curable composition according to a fifth aspect of the present invention further comprises (H) a filler in addition to the above components (A), (B) and (C) and/or the component (G) and the optional components (D), (E) and (F). The filler (H) used in the present invention is at least one filler selected from an inorganic filler and an organic composite filler. The shape of the filler used in the present invention may be spherical or amorphous and suitably selected together with a particulate filler. A filler having a porous structure or a hollow structure may be used without problems.

As the inorganic filler contained as the component (H) in the composition of the present invention may be used a known filler. Examples of the inorganic filler include the group I, II, III and IV elements of the periodic table, transition metals, oxides, hydroxides, chlorides, sulfates, sulfites, carbonates, oxo acid salts, phosphates and silicates thereof, and mixtures and composite salts thereof. More specific examples of the inorganic filler include glass fillers containing glass powders such as silicon dioxide, strontium glass, lanthanum glass and barium glass, quartz powders, barium sulfate, aluminum oxide, titanium oxide, barium salt, glass beads, glass fibers, barium fluoride, lead salt and talc, colloidal silica, silica gel, zirconium oxides, tin oxides carbon fibers, hydrotalcite compounds and other ceramic powders. Although the inorganic filler may be used as it is, it is preferably hydrophobized to increase the content of the inorganic filler in a cement by enhancing affinity between the polymerizable monomer (A) and the inorganic filler, or to produce an organic composite filler having high performance. A known surface treating agent for hydrophobization may be used, as exemplified by dialkyldichlorosilanes such as γ-(meth)acryloxypropyl trimethoxysilane, vinyl triethoxysilane, 3-aminopropylethoxysilane, 3-chloropropyltrimethoxsilane silylisocyanate, vinyl trichlorosilane, dimethyldichlorosilane and dioctyldichlorosilane, silane coupling agents such as hexamethylene disilazane, corresponding zirconium coupling agents and titanium coupling agents. As a surface treating method, a surface treating agent alone or a solution prepared by diluting a surface treating agent with an aqueous solution obtained by mixing together an organic solvent and water uniformly such as an ethanol aqueous solution is added to and mixed with an inorganic filler by means of a ball mill, twin-cylinder mixer or Henschel mixer and heated at 50 to 150 °C for several minutes to several hours (dry method). Or, an inorganic filler is added to an organic solvent such as ethanol or a solution obtained by mixing together an organic solvent and water uniformly such as an ethanol aqueous solution, or water to obtain a slurry, the above surface treating agent is added to the slurry so as to treat the inorganic filler at room temperature to reflux temperature for several minutes to several hours, the solvent is removed by a known method such as decantation or evaporation, and a heat treatment is carried out at 50 to 150°C for several hours (wet slurry method). Alternatively, a surface treating agent or the above aqueous solution is directly sprayed upon a high-temperature inorganic filler (spraying method). The inorganic filler should be treated by taking into consideration the properties of a silane treating agent and the inorganic filler. As a matter of course, a commercially available inorganic filler which has been surface treated may be used as it is or further surface treated by any one of the above methods. The above ethanol aqueous solution may be neutral or acidic. The surface treating agent is used in an amount of preferably 0. 1 to 60 parts by weight, more preferably 0.1 to 45 parts by weight, particularly preferably 0.1 to 30 parts by weight based on 100 parts by weight of the inorganic filler.

The organic composite filler used as the filler (H) in the present invention is obtained by coating the surface of the above-described inorganic filler with the polymerizable monomer through polymerization and grinding it. More specifically, particulate silica or zirconium oxide out of the above inorganic fillers is coated with the polymerizable monomer as the component (A) through polymerization, and the obtained polymer is ground. As a preferably used organic composite filler, an inorganic filler is coated with a polymerizable monomer containing trimethylolpropane tri(meth)acrylate (TMPT) as the main component through polymerization, and the obtained polymer is ground to obtain a filler (TMPT filler). These fillers may be used alone or in combination.

The median particle diameter of the filler (H) is preferably 0.001 to 100 μm, more preferably 0.005 to 50 μm, much more preferably 0.01 to 30 μm, particularly preferably 0.01 to 10 μm to provide suitable flowability to the dental curable composition of the present invention. Flowability can be also controlled by the shape of the filler, and a spherical filler is preferably used. To provide formability, a porous filler is more preferably used.

The dental curable composition according to the fifth aspect of the present invention is obtained by mixing the filler (H) with the above components (A), (B) and (C) and/or the component and the optional components (D), (E) and (F). The amount of the filler (H) is 1 to 400 parts by weight, preferably 50 to 250 parts by weight based on 100 parts by weight of the total of the components (A) and (B).

When the above first to fifth dental curable compositions described above contain an organic amine compound represented by the above formula (I) as a polymerization initiator, surprisingly, they are photopolymerized upon exposure to visible light without containing a photopolymerization initiator described above. Although the dental curable composition is photopolymerized upon exposure to visible light without containing a photopolymerization initiator as described above, it is also polymerized without containing a peroxide.

The dental curable composition according to a sixth aspect of the present invention further comprises (I) a sterilizing agent selected from components other than the above components (A) to (H) in addition to the components

of the above first to fifth dental curable compositions. The sterilizing agent as the component (I) which is contained in the sixth composition of the present invention is known as a sterilizing agent. It is used to enhance the disinfection property of an affected site and not particularly limited if it can kill or remove bacteria.

Examples of the sterilizing agent include cationic antibacterial agents such as benzalkonium chloride, benzethonium chloride, cetylpyridinium chloride, dericanium chrolide, chlorhexidine hydrochloride and gluconates including chlorhexidine gluconate; triclosan, isopropylmethylphenol, resorcin, ofloxacin, alexidine, hexetidine, iodine, potassium iodine, iodoform, povidone-iodine, fluorides such as fluorinated sodium, fluorinated tin and sodium monofluorophosphate, natural antibacterial agents such as thymol, menthol, eugenol, tannin, polyphenol, rhatany, chamomilla, myrrh, sage, tea extract, Japanese cypress extract, aloe extract, protamine, propolis and lysozyme, and antibiotics such as minocycline and tetracycline. At least one of these is preferably contained to enhance antibiotic and disinfecting properties.

The dental curable composition according to the sixth aspect of the present invention is obtained by mixing the sterilizing agent (I) with the components (A), (B) and (C) and/or the component (G) and the optional components (D), (E), (F), and (H). The amount of the sterilizing agent (I) is 0.01 to 200 parts by weight, preferably 0.1 to 150 parts by weight, more preferably 0.5 to 100 parts by weight based on 100 parts by weight of the total of the components (A) and (B).

The dental curable composition according to a seventh aspect of the present invention comprises a polymer other than the component (C) which dissolves in the component (A) and/or the component (G) as a component (J) selected from components other than the above components (A) to (I) in addition to the components of the first to sixth dental curable compositions. The component (J) contained in the seventh composition of the present invention is a polymer other than the component (C) which dissolves in the component (A) and/or the component (G) . Examples of the polymer include polymethylmethacrylate (PMMA), ethyl polymethylmethacrylate, propyl polymethacrylate, polybuthylmethacrylate, polyvinyl acetate (PVAc), polyethylene glycol (PEG), polypropylene glycol, polyvinyl alcohol (PVA) and polyvinyl pyrrolidone. When (meth)acrylic resin such as polymethylmethacrylate out of these is dissolved, the effect of increasing the degree of polymerization is obtained. Although the above polyvinyl acetate which is in the form of a high-molecular weight resin has low compatibility with the dental curable composition of the present invention or a polymerization cured product thereof, the polyvinyl acetate which is in the form of a low-molecular weight liquid is compatible with the dental curable composition or a cured product thereof and can dissolve in the dental curable composition and the cured product.

The dental curable composition according to the seventh aspect of the present invention is obtained by mixing the component (J), that is, a polymer other than the component (C) which dissolves in the component (A) and/or the component (G) with the above components (A), (B), (C) and/or the component (G) and the optional components (D), (E), (F), (H) and (I). The amount of the component (J) is 0.1 to 50 parts by weight, preferably 0.5 to 30 parts by weight, more preferably 1 to 10 parts by weight based on 100 parts by weight of the total of the components (A) and (B).

A known polymerization inhibitor may be contained in the composition in an amount of 1 to 5, 000 ppm to ensure the storage stability of the dental curable composition of the present invention. The polymerization inhibitor is not particularly limited but preferably a polymerization inhibitor or chain transfer agent for radically polymerizable monomers, as exemplified by hydroquinone, hydroquinone monomethyl ether, 2,6-di-t-butyl-p-cresol and 4-t-butyl catechol. The polymerization inhibitor can be used in limits that the curing of the present invention is not impeded, preferably 10 to 2,000 ppm.

A dye, pigment, thickener, organic acid, inorganic acid, hydroxide, ultraviolet absorbent, antistatic agent, surfactant and aroma chemical may be added to the dental curable composition of the present invention in addition to the above components as long as the effect of the present invention is not impaired.

Examples

The following examples are provided for the purpose of further illustrating the present invention but are in no way to be taken as limiting.

<marginal sealability test/pretreatment before removal test>

A bovine root tooth which had been frozen right before the test was used C:O expand the root canal for forming a columnar cavity having a diameter of 4 mm. After water contained in the expanded root canal was removed with an air gun, the inside of the root canal was dried with a paper point, and the composition of the present invention was filled into the root canal. It was left in a constant-temperature oven set at a relative humidity of 95 % and 37°C for 24 hours.

<marginal sealability test>

The bovine tooth which had been pretreated and left was cut at a right angle to the direction of the root canal with the ISOMET low-speed rotary diamond cutter to a thickness of 5 to 8 mm. The obtained sample was immersed in

a 5 % methylene blue aqueous solution for 1 hour and halved on a plane passing the center portion of the columnar cavity filled with the composition in a direction parallel to the root canal direction. As for marginal sealability, the degree of penetration of a pigment at the interface of the dentine when the composition was removed was judged. For the judgment of marginal sealability, a numerical value obtained by dividing the area into which the pigment penetrated by the total adhesion area was rounded off to two decimal places and classified as follows. ◎: 0, ○: ~0.20, △: 0.21 to 0.50, ×: ≧ 0.51 (0 means that the penetration of a pigment is not seen)

<removability test>

The composition filled in the bovine tooth which had been pretreated and left was removed by using a file, a broach or a resin moving device, and its removability was evaluated as "satisfactory" when it was easily removed. That is, when 4 or more out of 5 dental mechanics who have an experience of two or more years judge that the composition is easily removed, removability is judged as "satisfactory", when 3 to 2 dental mechanics judge that it is easily removed, removability is judged as "acceptable", and when 1 or less dental mechanic judges that it is easily removed, removability is judged as "unsatisfactory".

<X-ray contrast test>

This was carried out in accordance with ISO6876 (dental root canal sealing material).

<scanning electron microscope observation/elemental analysis/spectral analysis>

A removed human single-rooted tooth was used to expand the root canal with an engine reamer (#45 of Mannie Co., Ltd.) and washed with 15 % ethylenediamine tetraacetate (EDTA) and 2.5 % sodium hypochlorite (NaClO). After washing, water was removed with a paper point, and the composition was filled into the root canal by a single point method using a gutta-percha point (manufactured by Morita Co., Ltd.) and kept in a wet condition at 37°C for one night. Thereafter, the sample was cut in the tooth axis direction under water injection, mirror polished and treated with 6N hydrochloric acid and 1 % NaClO to observe the joint interface between the sealer and the gutta-percha through a scanning electron microscope (SEM, JEOL, JSM-5610LV). The elemental analysis of the joint interface between them was carried out with an energy dispersion type X-ray analyzing device (EDS, JEOL, JED-2200).

Symbols in the following examples and comparative examples denote the following substances.
4-MET: 4-methacryloyloxyethyltrimellitic acid
4-META: 4-methacryloyloxyethyltrimellitic anhydride HEMA: 2-hydroxyethyl methacrylate
3G: triethylene glycol dimethacrylate
9G: nonaethylene glycol dimethacrylate
14G: tetradecaethylene glycol dimethacrylate
23G: tricosaethylene glycol dimethacrylate
A-9300: ethoxylated triacrylate isocyanurate
GDMA: glycerin dimethacrylate
2.6E: 2,2-bis(4-methacryloyloxypolyethoxyphenyl)propane (average number of ethylene oxide recurring units: 2.6)
A-DPH: dipentaerythritol hexaacrylate
UDMA: 1,6-bis(methacryloxyethyloxycarbonylamino)-2,2,4-trimethylhexane
NPG-Na: sodium N-phenylglycine
p-TSNa: sodium p-toluenesulfinate
CQ: camphorquinone
DTMPO: 2,4,6-trimethylbenzoyldiphenylphosphine oxide $Bi_2(CO_3)O_2$: bismuth carbonate oxide (median particle diameter of 5 μm)
$CaWO_4$: calcium tungstate (median particle diameter of 4 μm)
$ZrO_2$: zirconium oxide (median particle diameter of 3 μm)
$SiO_2$: particulate silica (median particle diameter of 7 nm)
BPO: benzoyl peroxide
EVA: ethylene vinyl acetate copolymer (median particle diameter of 62 μm)
EPT: ethylene propylene terpolymer (median particle diameter of 320 μm)
PE: polyethylene (median particle diameter of 6 μm)
PEG: polyethylene glycol (weight average molecular weight of 500,000)
PMMA: polymethylmethacrylate (number average molecular weight of 40,000, weight average molecular weight of 400,000, median particle diameter of 40 μm)
PVAc: polyvinyl acetate (median particle diameter of 630 μm)

Examples 1 to 19

Components ((A) to (J)) shown in Table 1 were collected in a ratio shown in Table 1 to prepare the dental curable compositions of the present invention. Since the weight ratio was calculated based on weighed values by rounding to the significant digit in Table 1, the weight ratio value had a rounding error. The marginal sealability test, removability test and X-ray contrast test of the compositions, the scanning electron microscope (SEM) observation of the joint interfaces and the elemental analysis of the joint interfaces were carried out.

As a result, all the samples showed a good result at 0 to 0.2 in the marginal sealability test. All the samples were judged as "acceptable" or "satisfactory" and there was no sample which was judged as "unsatisfactory" in the removability test. All the samples had not less than 300 %/Al in the X-ray contrast test. Further, as a result of the SEM observation of the joint interface, there was no crack at the joint interface in all the examples, and it was confirmed that the composition was well bonded and that a layer impregnated with the composition was seen in the dentine and also the gutta-percha point.

In the analysis of these layers, elements derived from the constituent components of the composition and the gutta-percha were detected by the EDS analysis of the joint interface (composition/gutta-percha point) so that an intermediate layer seen between the gutta-percha point and the dental composition could be judged as "a layer in which the constituent components of the composition were impregnated into the gutta-percha point". An example of this result (Example 11) is shown in Fig. 1 and Fig. 2.

Fig. 1 shows an SEM image of the joint interface between the gutta-percha point and the composition. It was confirmed that there was a 10 μm-thick intermediate layer at the joint interface. When linear analysis was carried out in EDS analysis (Fig. 2), no Si element derived from the composition was seen in this intermediate layer, and it was made clear that the amount of Zn derived from the gutta-percha point and the amount of C derived from the composition changed in slope. It was concluded from this that the intermediate layer was formed by the infiltration of the composition into the gutta-percha point and the polymerization and curing of the composition.

Comparative Examples 1 and 2

Components ((A) to (H)) shown in Table 2 were collected in a ratio shown in Table 2 to prepare compositions. The marginal sealability test, removability test and X-ray contrast test were carried out by using these compositions. As a result, Comparative Example 1 was excellent at 0 in marginal sealability but unsatisfactory in terms of removability. Comparative Example 2 was "satisfactory" in terms of removability but unsatisfactory at 0.51 or more in terms of marginal sealability.

Table 1

| No. | Dental curable composition (parts by weight) | | | | |
|---|---|---|---|---|---|
| | (A) | (B) | (C) | (D) | (E) |
| Example 1 | HEMA/23G/A-9300/4-MET (42.4/18.5/9.6/19.4) | NPG-Na (10.1) | EVA (66.9) | $FeCl_3$ (0.009) | — |
| Example 2 | HEMA/23G/3G/A-9300/4-MET (39.0/15.2/6.7/9.6/19.4) | NPG-Na (10.1) | EVA (66.9) | $FeCl_3$ (0.009) | — |
| Example 3 | HEMA/23G/A-9300/4-MET (38.3/20.1/10.7/21.2) | NPG-Na (9.7) | EVA (63.8) | $FeCl_3$ (0.009) | — |
| Example 4 | HEMA/23G/3G/A-9300/4-MET (35.1/16.9/6.4/10.7/21.2) | NPG-Na (9.7) | EVA (63.8) | $FeCl_3$ (0.009) | — |
| Example 5 | HEMA/GDMA/2.6E/A-9300/4-MET (18.5/18.2/13.5/22.9/18.2) | NPG-Na (8.7) | EVA (57.4) | — | p-TSNa (0.75) |
| Example 6 | HEMA/GDMA/2.6E/A-9300/4-MET (18.5/18.2/13.5/22.9/18.2) | NPG-Na (8.7) | EPT (60.5) | — | p-TSNa (0.75) |
| Example 7 | HEMA/GDMA/2.6E/A-9300/4-MET (18.3/18.2/13.5/23.0/18.2) | NPG-Na (8.8) | EVA (57.5) | — | p-TSNa (0.75) |
| Example 8 | HEMA/GDMA/2.6E/A-9300/4-MET (18.5/18.2/13.5/22.9/18.2) | NPG-Na (8.7) | EVA (57.4) | — | p-TSNa (0.75) |
| Example 9 | HEMA/9G/A-9300/4-MET (28.8/33.3/10.4/19.5) | NPG-Na (8.0) | EVA (74.7) | — | — |

Table 1 (continued)

| No. | Dental curable composition (parts by weight) | | | | |
|---|---|---|---|---|---|
| | (A) | (B) | (C) | (D) | (E) |
| Example 10 | HEMA/23G/A-9300/4-MET (29.0/33.0/10.0/20.0) | NPG-Na (8.0) | EVA (75.0) | — | — |
| Example 11 | HEMA/23G/A-9300/4-MET (30.9/35.8/11.1/21.0) | NPG-Na (1.2) | EVA (75.2) | — | — |
| Example 12 | HEMA/23G/A-9300/4-MET (28.7/33.3/10.5/19.3) | NPG-Na (8.2) | — | — | — |
| Example 13 | HEMA/23G/A-9300/4-MET (30.1/33.7/10.9/20.5) | NPG-Na/CQ (3.6/1.2) | EPT (78.3) | — | p-TSNa (1.2) |
| Example 14 | HEMA/23G/A-DPH/4-META (30.1/33.7/10.9/20.5) | NPG-Na/DTMPO (3.6/1.2) | EPT (78.3) | — | p-TSNa (1.2) |
| Example 15 | HEMA/23G/A-9300/4-MET (34.5/34.6/9.1/9.1) | NPG-Na (12.7) | EVA (118.2) | — | — |
| Example 16 | HEMA/23G/14G/A-9300/4-MET (30.7/17.2/18.4/11.0/20.9) | NPG-Na (1.8) | PE (61.3) | — | — |
| Example 17 | HEMA/23G/UDMA/4-MET (40.8/26.4/10.8/18.0) | NPG-Na (4.0) | PE (52.5) | — | — |
| Example 18 | HEMA/23G/UDMA/4-MET (41.6/27.0/11.0/18.4) | NPG-Na (2.0) | PE (66.3) | — | — |
| Example 19 | HEMA/23G/UDMA/4-MET (41.6/27.0/11.0/18.4) | NPG-Na (2.0) | PE (66.0) | — | p-TSNa (0.7) |
| Comparative Example 1 | HEMA/GDMA/A-9300/2.6E/4-MET (20.0/18.0/23.0/14.0/18.0) | NPG-Na (7.0) | — | — | p-TSNa (1.0) |
| Comparative Example 2 | 9G/23G (9.4/84.1) | BPO/NPG-Na (0.9/5.6) | — | — | — |

Table 1 (continued)

| No. | Dental curable composition (parts by weight) | | | | |
|---|---|---|---|---|---|
| | (F) | (G) | (H) | (I) | (J) |
| Example 1 | — | $H_2O$ (21.6) | $Bi_2(CO_3)O_2/SiO_2$ (89.0/3.4) | — | — |
| Example 2 | — | $H_2O$ (21.6) | $Bi_2(CO_3)O_2/SiO_2$ (89.0/3.4) | — | — |
| Example 3 | — | $H_2O$ (16.1) | $Bi_2(CO_3)O_2/SiO_2$ (85.0/3.2) | — | — |
| Example 4 | — | $H_2O$ (16.1) | $Bi_2(CO_3)O_2/SiO_2$ (85.0/3.2) | — | — |
| Example 5 | — | $H_2O$ (4.7) | $Bi_2(CO_3)O_2$ (86.5) | — | — |
| Example 6 | — | $H_2O$ (4.7) | $CaWO_4$ (86.5) | — | — |
| Example 7 | Citric acid (0.25) | $H_2O$ (4.7) | $Bi_2(CO_3)O_2$ (86.8) | — | — |
| Example 8 | — | $H_2O$ (4.7) | — | $CHI_3$ (86.5) | — |
| Example 9 | — | $H_2O$ (23.0) | $Bi_2(CO_3)O_2$ (106.9) | — | — |

Table 1 (continued)

| No. | Dental curable composition (parts by weight) | | | | |
|---|---|---|---|---|---|
| | (F) | (G) | (H) | (I) | (J) |
| Example 10 | — | $H_2O$ (23.0) | $Bi_2(CO_3)O_2$ (107.0) | — | — |
| Example 11 | — | $H_2O$ (24.7) | $Bi_2(CO_3)O_2/SiO_2$ (122.2/12.0) | — | — |
| Example 12 | — | $H_2O$ (22.8) | $Bi_2(CO_3)O_2$ (108.8) | — | PEG (2.3) |
| Example 13 | — | $H_2O$ (24.1) | $Bi_2(CO_3)O_2$ (115.7) | — | — |
| Example 14 | — | $H_2O$ (35.7) | $Bi_2(CO_3)O_2$ (151.7) | $CHI_3$ (0.5) | PMMA/PVAc (1.5/1.5) |
| Example 15 | — | $H_2O$ (85.5) | $Bi_2(CO_3)O_2/ZrO_2/SiO_2$ (156.7/10.4/2.0) | — | PEG (9.1) |
| Example 16 | | $H_2O$ (24.5) | $Bi_2(CO_3)O_2$ (90.2) | — | — |
| Example 17 | — | $H_2O$ (24.0) | $Bi_2(CO_3)O_2$ (75.5) | — | — |
| Example 18 | — | $H_2O$ (24.5) | $Bi_2(CO_3)O_2$ (66.3) | — | — |
| Example 19 | — | $H_2O$ (24.5) | $Bi_2(CO_3)O_2$ (66.0) | — | — |
| Comparative Example 1 | — | $H_2O$ (3.0) | $ZrO_2/SiO_2$ (77.0/12.0) | — | — |
| Comparative Example 2 | — | — | $Bi_2(CO_3)O_2$ (275.0) | — | — |

Table 2

| No. | Marginal sealability | Removability | X-ray contrast (%/Al) |
|---|---|---|---|
| Example 1 | ◎ | Satisfactory | 420 |
| Example 2 | ◎ | Satisfactory | 406 |
| Example 3 | ◎ | Satisfactory | 411 |
| Example 4 | ◎ | Satisfactory | 393 |
| Example 5 | ◎ | Acceptable | 450 |
| Example 6 | ◎ | Satisfactory | 317 |
| Example 7 | ◎ | Acceptable | 432 |
| Example 8 | ◎ | Satisfactory | 324 |
| Example 9 | ◎ | Satisfactory | 486 |
| Example 10 | ◎ | Satisfactory | 480 |
| Example 11 | ◎ | Acceptable | 516 |
| Example 12 | ○ | Acceptable | 481 |
| Example 13 | ◎ | Satisfactory | 499 |
| Example 14 | ○ | Satisfactory | 527 |
| Example 15 | ○ | Satisfactory | 531 |
| Example 16 | ◎ | Satisfactory | 433 |
| Example 17 | ◎ | Satisfactory | 413 |
| Example 18 | ◎ | Satisfactory | 405 |
| Example 19 | ◎ | Satisfactory | 398 |
| Comparative Example 1 | ◎ | Unsatisfactory | 380 |
| Comparative Example 2 | × | Satisfactory | 753 |

The dental curable composition of the present invention has excellent marginal sealability even for a tooth which is moist and not pretreated through a simple operation and excellent removability. Further, the dental curable composition also has excellent sealing characteristic when a layer impregnated with the dental curable composition is formed at the joint interface with the dentine and the gutta-percha interface, thereby making it possible to carry out dental treatment extremely effectively.

Effect of the Invention

The dental curable composition of the present invention has the high permeability of a polymerizable monomer component into the inside of the dentine from a moist tooth surface and excellent adhesion to the dentine which is not subjected to a surface treatment as polymerization starts from the direction of the moist dentine interface, and does not crack the adhesive interface. Therefore, the invasion of bacteria into the oral cavity at a repair site can be suppressed effectively. There can be provided a dental curable composition which is extremely useful as a resin material for repairing dental caries in a cervical portion by providing flexibility and abrasion resistance due to an effect obtained by containing the long-chain polymerizable monomer having a chain length of 19 or more atoms (A') and containing water alone or a mixed solvent water and an organic solvent miscible with water, which is (G) a solvent miscible with the long-chain polymerizable monomer in the composition and/or mixing the soft resin component (C) being not substantially dissolved by the component (A) and having median particle diameter of 0.01 to 500 $\mu$m. Further, there can be provided a dental curable composition which is extremely useful as a repair material such as a root canal sealer, a root canal filling sealer, a temporary sealing material or a temporary luting material as it is easily removed when it must be removed though it has excellent marginal sealability.

Patent literature

US 2002/0198284
EP 1 695685
US 2009/0227699
JP 2000/143432
JP 2008/024668
JP 2005/179282
JP 2008/285645
JP 2008/308418
JP S6 317 816
Each one of the above-mentioned documents discloses a polymerizable dental composition comprising a polymerizable monomer and an organic amine-based polymerization initiator.

## Claims

**1.** A dental curable composition comprising:

(A) a polymerizable monomer,
(B) an organic amine-based polymerization initiator, and
(C) a soft resin material being not dissolved by the component (A) and having a durometer A hardness, measured by a type A durometer based on JIS K6253, of not more than 90 and median particle diameter of 0.01 to 500 μm, the soft resin material (C) being at least one selected from the group consisting of gutta-percha, polyethylene, polypropylene, ethylene propylene copolymer, ethylene propylene terpolymer, silicone polymer, polyisoprene, ethylene vinyl acetate copolymer and acrylic acid ester copolymer,
wherein
the component (A) contains

- (A') a long-chain polymerizable monomer having a chain length of 19 or more atoms and containing a polyalkylene glycol di(meth)acrylate having at least 4 oxyalkylene recurring units ($-(-(-CH_2-)_p-O-)_n-$), wherein p is an integer of 2 or more, and n is an integer of 4 or more, in the molecule and
- a polymerizable monomer having an acidic group in the molecule,

the composition contains water alone or a mixed solvent of water and an organic solvent miscible with water, which is (G) a solvent miscible with the long-chain polymerizable monomer, and
when

the amount (parts by weight) based on 100 parts by weight of the component (A) of the component (A') is represented by [a'] and
the amounts (parts by weight) based on 100 parts by weight of the total of the components (A) and (B) of the components (A), (B) and (C) are represented by (a), (b) and (c), respectively,

$$70 \leqq (a) \leqq 99.99,$$

$$0.01 \leqq (b) \leqq 30,$$

$$1 \leqq [a']/5 + (c),$$

$$0 \leqq [a'] \leqq 95,$$

and

$$0 < (c) \leqq 250.$$

2. The dental curable composition according to claim 1, wherein the polymerizable monomer (A) contains a polymerizable monomer having at least one acid group selected from the group consisting of carboxylic acid group, phosphoric acid group, thiophosphoric acid group, sulfonic acid group, pyrophosphoric acid group and sulfinic acid group.

3. The dental curable composition according to claim 1 or 2, wherein the polymerizable monomer (A) contains a polymerizable monomer having at least one hydroxyl group.

4. The dental curable composition according to claim 1, wherein the polymerizable monomer (A') has at least two polymerizable groups.

5. The dental curable composition according to claim 1, wherein the long-chain polymerizable monomer (A') is polyethylene glycol di(meth)acrylate and/or polypropylene glycol di(meth)acrylate having 4 to 60 recurring units derived from propylene glycol and/or ethylene glycol.

6. The dental curable composition according to any one of claims 1 to 3, wherein the polymerizable monomer (A) contains a polymerizable polyfunctional (meth)acrylate having at least 3 ethylenically unsaturated bonds in the molecule.

7. The dental curable composition according to any one of claims 1 to 3 and 6, wherein the polymerizable monomer (A) contains a polymerizable monomer having a triazine ring derivative group.

8. The dental curable composition according to any one of claims 1 to 3, 6 and 7, wherein the polymerizable monomer (A) contains a dipentaerythritol-based polymerizable monomer.

9. The dental curable composition according to any one of claims 1 to 8, wherein the organic amine-based polymerization initiator (B) contains an organic amine compound represented by the following formula (I) or (II).

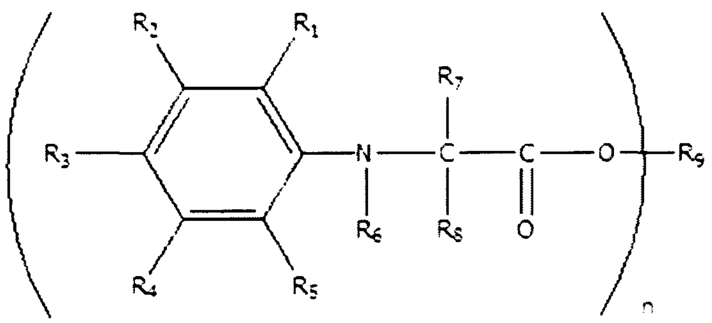

(I)

(wherein, $R_1$, $R_2$, $R_3$, $R_4$ and $R_5$ are each independently a hydrogen atom, alkyl group, aryl group, aryloxy group, alkoxyl group, nitro group, acyl group, acyloxy group, hydroxyl group or halogen atom, $R_6$ is a hydrogen atom, alkyl group or aryl group, $R_7$ and $R_8$ are each independently a hydrogen atom or alkyl group, $R_9$ is a hydrogen atom or metal atom, and n is 1 when $R_9$ is hydrogen and the same integer as the valence of a metal atom when $R_9$ is the metal atom.)

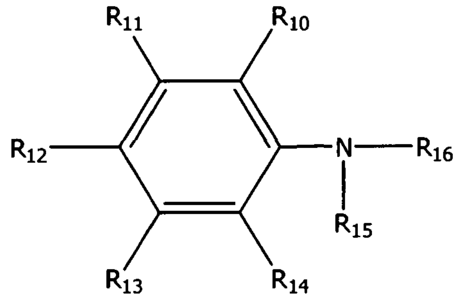

(II)

(wherein, $R_{10}$, $R_{11}$, $R_{12}$, $R_{13}$ and $R_{14}$ are each independently a hydrogen atom, alkyl group, aryl group, aryloxy group, alkoxyl group, nitro group, acyl group, acyloxy group, hydroxyl group or halogen atom, and $R_{15}$ and $R_{16}$ are each independently a hydrogen atom, alkyl group, aryl group or substituted alkyl group having a hetero atom.)

**10.** The dental curable composition according to any one of claims 1 to 9, wherein the soft resin material (C) has a durometer D hardness of not more than 60.

**11.** The dental curable composition according to any one of claims 1 to 10 which further comprises (D) a transition metal compound.

**12.** The dental curable composition according to claim 11,
wherein the content of the component (D) is 0.001 to 10 parts by weight based on 100 parts by weight of the total of the components (A) and (B).

**13.** The dental curable composition according to any one of claims 1 to 12 which further comprises (E) a sulfur-containing reducing compound.

**14.** The dental curable composition according to claim 13,
wherein the content of the component (E) is 0.01 to 10 parts by weight based on 100 parts by weight of the total of the components (A) and (B).

**15.** The dental curable composition according to any one of claims 1 to 14 which further comprises (F) a hydroxylic acid compound.

**16.** The dental curable composition according to claim 15,
wherein the content of the hydroxylic acid compound (F) is 0.01 to 10 parts by weight based on 100 parts by weight of the total of the components (A) and (B).

**17.** The dental curable composition according to any one of claims 1 to 16 which further comprises a peroxide in an amount of not more than 0.1 part by weight based on 100 parts by weight of total of the components (A) and (B).

**18.** The dental curable composition according to claim 1,
wherein the content of the solvent (G) is 0.1 to 300 parts by weight based on 100 parts by weight of the total of the components (A) and (B).

**19.** The dental curable composition according to any one of claims 1 to 18 which further comprises (H) at least one filler selected from an inorganic filler and an organic composite filler.

**20.** The dental curable composition according to claim 19,
wherein the content of the filler (H) is 1 to 400 parts by weight based on 100 parts by weight of the total of the components (A) and (B).

**21.** The dental curable composition according to claim 19,
wherein the inorganic filler (H) is at least one selected from the group consisting of zirconium oxide, zinc oxide, barium sulfate, bismuth oxide, bismuth oxychloride, bismuth carbonate oxide and calcium tungstate.

**22.** The dental curable composition according to any one of claims 1 to 21which further comprises (I) a sterilizing agent.

**23.** The dental curable composition according to claim 22, wherein the sterilizing agent (I) is at least one selected from the group consisting of benzalkonium chloride, benzethonium chloride, isopropylmethylphenol, cetylpyridinium chloride, resorcin, chlorhexidine hydrochloride, chlorhexidine gluconate, iodine, potassium iodide, povidone-iodine and iodoform.

**24.** The dental curable composition according to claim 22 or 23, wherein the content of the sterilizing agent (I) is 0.01 to 200 parts by weight based on 100 parts by weight of the total of the components (A) and (B).

**25.** The dental curable composition according to any one of claims 1 to 24 which further comprises (J) a polymer other than the component (C) which dissolves in the component (A) and/or the component (G).

**26.** The dental curable composition according to claim 25, wherein the content of the polymer (J) is 0.1 to 50 parts by weight based on 100 parts by weight of the total of the components (A) and (B).

**27.** The dental curable composition according to claim 5, wherein the long-chain polymerizable monomer (A') is polyethylene glycol di(meth)acrylate and/or polypropylene glycol di(meth)acrylate having 11 to 28 recurring units derived from propylene glycol and/or ethylene glycol.

**28.** The dental curable composition of any one claims 1 to 26 for use in dental medicine as a root canal filling material, lining material, temporary sealing material or temporary luting material.

**Patentansprüche**

**1.** Härtbare Dentalzusammensetzung, umfassend:

(A) ein polymerisierbares Monomer,
(B) einen Polymerisationsinitiator auf Basis eines organischen Amins und
(C) ein Weichharzmaterial, das durch die Komponente (A) nicht aufgelöst wird und eine Durometer-A-Härte, gemessen mit einem Durometer vom Typ A, basierend auf JIS K6253, von nicht mehr als 90 und einen medianten Partikeldurchmesser von 0,01 bis 500 μm besitzt, wobei das Weichharzmaterial (C) wenigstens eines ist, ausgewählt aus der Gruppe, bestehend aus
Guttapercha, Polyethylen, Polypropylen, Ethylen-Propylen-Copolymer, Ethylen-Propylen-Terpolymer, Siliconpolymer, Polyisopren, Ethylen-Vinylacetat-Copolymer und Acrylsäureestercopolymer,
wobei
die Komponente (A) das folgende enthält:

- (A') ein langkettiges polymerisierbares Monomer, das eine Kettenlänge von 19 oder mehr Atomen hat und ein Polyalkylengly-coldi(meth)acrylat mit wenigstens 4 Oxyalkylen-Wiederholungseinheiten ($-(-(-CH_2-)_p-O-)_n-$), worin p eine ganze Zahl von 2 oder mehr ist und n eine ganze Zahl von 4 oder mehr ist, in dem Molekül enthält, und
- ein polymerisierbares Monomer mit einer sauren Gruppe in dem Molekül,

die Zusammensetzung Wasser alleine oder ein gemischtes Lösungsmittel aus Wasser und einem organischen mit Wasser mischbaren Lösungsmittel, das (G) ein mit dem langkettigen polymerisierbaren Monomer mischbares Lösungsmittel ist, enthält, und wenn
die Menge (Gewichtsteile), basierend auf 100 Gewichtsteilen der Komponente (A) der Komponente (A') durch [a'] dargestellt ist und
die Mengen (Gewichtsteile), basierend auf 100 Gewichtsteilen der Gesamtheit der Komponenten (A) und (B), der Komponenten (A) und (B) und (C) durch (a), (b) bzw. (c) dargestellt sind,

$$70 \leqq (a) \leqq 99{,}99,$$

$$0{,}01 \leqq (b) \leqq 30,$$

$$1 \leqq [a']/5 + (c),$$

$$0 \leqq [a'] \leqq 95$$

und

$$0 < (c) \leqq 250.$$

**2.** Härtbare Dentalzusammensetzung gemäß Anspruch 1, wobei das polymerisierbare Monomer (A) ein polymerisierbares Monomer mit wenigstens einer Säuregruppe, ausgewählt aus der Gruppe, bestehend aus einer Carbonsäuregruppe, einer Phosphorsäuregruppe, einer Thiophosphorsäuregruppe, einer Sulfonsäuregruppe, einer Pyrophosphorsäuregruppe und einer Sulfinsäuregruppe, enthält.

**3.** Härtbare Dentalzusammensetzung gemäß Anspruch 1 oder 2, wobei das polymerisierbare Monomer (A) ein polymerisierbares Monomer mit wenigstens einer Hydroxylgruppe enthält.

**4.** Härtbare Dentalzusammensetzung gemäß Anspruch 1, wobei das polymerisierbare Monomer (A') wenigstens zwei polymerisierbare Gruppen hat.

**5.** Härtbare Dentalzusammensetzung gemäß Anspruch 1, wobei das langkettige polymerisierbare Monomer (A') Polyethylengly-coldi(meth)acrylat und/oder Polypropylenglycoldi(meth)acrylat mit 4 bis 60 Wiederholungseinheiten, die von Propylenglycol und/oder Ethylenglycol abgeleitet sind, ist.

**6.** Härtbare Dentalzusammensetzung gemäß einem der Ansprüche 1 bis 3, wobei das polymerisierbare Monomer (A) ein polymerisierbares polyfunktionelles (Meth)acrylat mit wenigstens 3 ethylenisch ungesättigten Bindungen in dem Molekül enthält.

**7.** Härtbare Dentalzusammensetzung gemäß einem der Ansprüche 1 bis 3 und 6, wobei das polymerisierbare Monomer (A) ein polymerisierbares Monomer mit einer Triazinringderivatgruppe enthält.

**8.** Härtbare Dentalzusammensetzung gemäß einem der Ansprüche 1 bis 3, 6 und 7, wobei das polymerisierbare Monomer (A) ein polymerisierbares Monomer auf Dipentaerythritbasis enthält.

**9.** Härtbare Dentalzusammensetzung gemäß einem der Ansprüche 1 bis 8, wobei der Polymerisationsinitiator auf Basis eines organischen Amins (B) eine organische Aminverbindung, die durch die folgende Formel (I) oder (II) dargestellt ist, enthält,

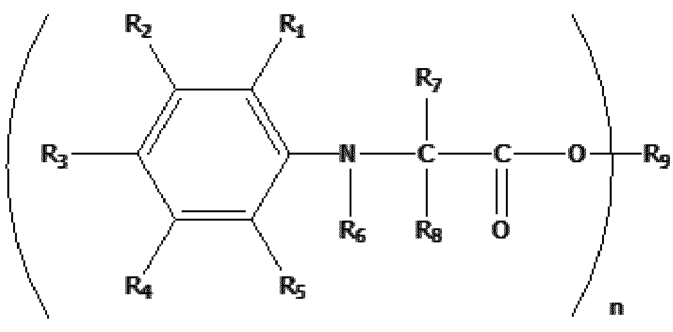

(I)

(worin $R_1$, $R_2$, $R_3$, $R_4$ und $R_5$ jeweils unabhängig ein Wasserstoffatom, eine Alkylgruppe, eine Arylgruppe, eine Aryloxygruppe, eine Alkoxylgruppe, eine Nitrogruppe, eine Acylgruppe, eine Acyloxygruppe, eine Hydroxylgruppe oder ein Halogenatom sind, $R_6$ ein Wasserstoffatom, eine Alkylgruppe oder Arylgruppe ist, $R_7$ und $R_8$ jeweils unabhängig ein Wasserstoffatom oder eine Alkylgruppe sind, $R_9$ ein Wasserstoffatom oder ein Metallatom ist, und n

1 ist, wenn $R_9$ Wasserstoff ist, und die gleiche ganze Zahl wie die Valenz des Metallatoms ist, wenn $R_9$ das Metallatom ist.)

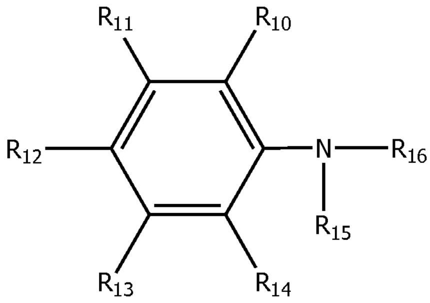

(II)

(worin $R_{10}$, $R_{11}$, $R_{12}$, $R_{13}$ und $R_{14}$ jeweils unabhängig ein Wasserstoffatom, eine Alkylgruppe, eine Arylgruppe, eine Aryloxygruppe, eine Alkoxylgruppe, eine Nitrogruppe, eine Acylgruppe, eine Acyloxygruppe, eine Hydroxylgruppe oder ein Halogenatom sind, und $R_{15}$ und $R_{16}$ jeweils unabhängig ein Wasserstoffatom, eine Alkylgruppe, eine Arylgruppe oder eine substituierte Alkylgruppe mit einem Heteroatom sind.)

**10.** Härtbare Dentalzusammensetzung gemäß einem der Ansprüche 1 bis 9, wobei das Weichharzmaterial (C) eine Durometer-D-Härte von nicht mehr als 60 hat.

**11.** Härtbare Dentalzusammensetzung gemäß einem der Ansprüche 1 bis 10, die weiterhin (D) eine Übergangsmetallverbindung umfasst.

**12.** Härtbare Dentalzusammensetzung gemäß Anspruch 11, wobei der Gehalt der Komponente (D) 0,001 bis 10 Gewichtsteile, basierend auf 100 Gewichtsteilen der Gesamtheit der Komponenten (A) und (B), ist.

**13.** Härtbare Dentalzusammensetzung gemäß einem der Ansprüche 1 bis 12, die weiterhin (E) eine schwefelhaltige reduzierende Verbindung umfasst.

**14.** Härtbare Dentalzusammensetzung gemäß Anspruch 13, wobei der Gehalt der Komponente (E) 0,01 bis 10 Gewichtsteile, basierend auf 100 Gewichtsteilen der Gesamtheit der Komponenten (A) und (B), ist.

**15.** Härtbare Dentalzusammensetzung gemäß einem der Ansprüche 1 bis 14, die weiterhin (F) eine Hydroxylsäureverbindung umfasst.

**16.** Härtbare Dentalzusammensetzung gemäß Anspruch 15, wobei der Gehalt der Hydroxylsäureverbindung (F) 0,01 bis 10 Gewichtsteile, basierend auf 100 Gewichtsteilen der Gesamtheit der Komponenten (A) und (B), ist.

**17.** Härtbare Dentalzusammensetzung gemäß einem der Ansprüche 1 bis 16, die weiterhin ein Peroxid in einer Menge von nicht mehr als 0,1 Gewichtsteilen, basierend auf 100 Gewichtsteilen der Gesamtheit der Komponenten (A) und (B), umfasst.

**18.** Härtbare Dentalzusammensetzung gemäß Anspruch 1, wobei der Gehalt des Lösungsmittels (G) 0,1 bis 300 Gewichtsteile, basierend auf 100 Gewichtsteilen der Gesamtheit der Komponenten (A) und (B), ist.

**19.** Härtbare Dentalzusammensetzung gemäß einem der Ansprüche 1 bis 18, die weiterhin (H) wenigstens einen Füllstoff, ausgewählt aus einem anorganischen Füllstoff und einem organischen Kompositfüllstoff, umfasst.

**20.** Härtbare Dentalzusammensetzung gemäß Anspruch 19, wobei der Gehalt des Füllstoffs (H) 1 bis 400 Gewichtsteile, basierend auf 100 Gewichtsteilen der Gesamtheit der Komponenten (A) und (B), ist.

**21.** Härtbare Dentalzusammensetzung gemäß Anspruch 19, wobei der anorganische Füllstoff (H) wenigstens einer ist, ausgewählt aus der Gruppe, bestehend aus Zirconiumoxid, Zinkoxid, Bariumsulfat, Bismuthoxid, Bismuthoxychlorid, Bismuthcarbonatoxid und Calciumwolframat.

**22.** Härtbare Dentalzusammensetzung gemäß einem der Ansprüche 1 bis 21, die weiterhin (I) ein Sterilisierungsmittel

umfasst.

**23.** Härtbare Dentalzusammensetzung gemäß Anspruch 22, wobei das Sterilisierungsmittel (I) wenigstens eines ist, ausgewählt aus der Gruppe, bestehend aus Benzalkoniumchlorid, Benzethoniumchlorid, Isopropylmethylphenol, Cetylpyridiniumchlorid, Resorcin, Chlorhexidinhydrochlorid, Chlorhexidingluconat, Iod, Kaliumiodid, Povidon-Iod und Iodoform.

**24.** Härtbare Dentalzusammensetzung gemäß Anspruch 22 oder 23, wobei der Gehalt des Sterilisierungsmittels (I) 0,01 bis 200 Gewichtsteile, basierend auf 100 Gewichtsteilen der Gesamtheit der Komponenten (A) und (B), ist.

**25.** Härtbare Dentalzusammensetzung gemäß einem der Ansprüche 1 bis 24, die weiterhin (J) ein Polymer anders als die Komponente (C), das sich in der Komponente (A) und/oder der Komponente (G) löst, umfasst.

**26.** Härtbare Dentalzusammensetzung gemäß Anspruch 25, wobei der Gehalt des Polymers (J) 0,1 bis 50 Gewichtsteile, basierend auf 100 Gewichtsteilen der Gesamtheit der Komponenten (A) und (B), ist.

**27.** Härtbare Dentalzusammensetzung gemäß Anspruch 5, wobei das langkettige polymerisierbare Monomer (A') Polyethylengly-coldi(meth)acrylat und/oder Polypropylenglycoldi(meth)acrylat mit 11 bis 28 Wiederholungseinheiten, die von Propylenglycol und/oder Ethylenglycol abgeleitet sind, ist.

**28.** Härtbare Dentalzusammensetzung gemäß einem der Ansprüche 1 bis 26 zur Verwendung in der Zahnmedizin als ein Wurzelkanalfüllungsmaterial, Überzugsmaterial, temporäres Abdichtungsmaterial oder temporäres Kittmaterial.

## Revendications

**1.** Composition dentaire durcissable comprenant :

(A) un monomère polymérisable,
(B) un initiateur de polymérisation à base d'amine organique, et
(C) un matériau de résine souple qui n'est pas dissous par le composant (A) et présentant une dureté A d'après duromètre, mesurée par un duromètre de type A sur la base de la norme JIS K6253, d'au plus 90 et un diamètre particulaire médian de 0,01 à 500 μm, le matériau de résine souple (C) étant au moins l'un sélectionné dans le groupe constitué de la guttapercha, d'un polyéthylène, d'un polypropylène, d'un copolymère d'éthylène et de propylène, d'un terpolymère d'éthylène et de propylène, d'un polymère de silicone, d'un polyisoprène, d'un copolymère d'éthylène et d'acétate de vinyle et d'un copolymère d'ester d'acide acrylique,
dans laquelle
le composant (A) contient

- (A') un monomère polymérisable à longue chaîne ayant une longueur de chaîne de 19 atomes ou plus et contenant un di(méth)acrylate de polyalkylène glycol ayant au moins 4 motifs récurrents d'oxyalkylène ($-(-(-CH_2-)_p-O-)_n-$), dans laquelle p est un nombre entier égal à 2 ou plus, et n est un nombre entier égal à 4 ou plus, dans la molécule et
- un monomère polymérisable ayant un groupe acide dans la molécule,

la composition contient seulement de l'eau ou un solvant composé d'eau et d'un solvant organique miscible à l'eau, qui est (G) un solvant miscible avec le monomère polymérisable à longue chaîne, et
quand
la quantité (parties en poids) sur la base de 100 parties en poids du composant (A) du composant (A') est représentée par [a'] et
les quantités (parties en poids) sur la base de 100 parties en poids du total des composants (A) et (B) des composants (A), (B) et (C) sont représentées par (a), (b) et (c), respectivement,

$$70 \leq (a) \leq 99{,}99,$$

$$0{,}01 \leq (b) \leq 30,$$

$$1 \leq [a']/5 + (c),$$

$$0 \leq [a'] \leq 95,$$

et

$$0 < (c) \leq 250.$$

**2.** Composition dentaire durcissable selon la revendication 1, dans laquelle le monomère polymérisable (A) contient un monomère polymérisable ayant au moins un groupe acide sélectionné dans le groupe constitué d'un groupe acide carboxylique, d'un groupe acide phosphorique, d'un groupe acide thiophosphorique, d'un groupe acide sulfonique, d'un groupe acide pyrophosphorique et d'un groupe acide sulfinique.

**3.** Composition dentaire durcissable selon la revendication 1 ou 2, dans laquelle le monomère polymérisable (A) contient un monomère polymérisable ayant au moins un groupe hydroxyle.

**4.** Composition dentaire durcissable selon la revendication 1, dans laquelle le monomère polymérisable (A') a au moins deux groupes polymérisables.

**5.** Composition dentaire durcissable selon la revendication 1, dans laquelle le monomère polymérisable à longue chaîne (A') est un di(méth)acrylate de polyéthylène glycol et/ou un di(méth)acrylate de polypropylène glycol ayant 4 à 60 motifs récurrents dérivés du propylène glycol et/ou de l'éthylène glycol.

**6.** Composition dentaire durcissable selon l'une quelconque des revendications 1 à 3, dans laquelle le monomère polymérisable (A) contient un (méth)acrylate polyfonctionnel polymérisable ayant au moins 3 liaisons à insaturation éthylénique dans la molécule.

**7.** Composition dentaire durcissable selon l'une quelconque des revendications 1 à 3 et 6, dans laquelle le monomère polymérisable (A) contient un monomère polymérisable ayant un groupe dérivé d'un cycle triazine.

**8.** Composition dentaire durcissable selon l'une quelconque des revendications 1 à 3, 6 et 7, dans laquelle le monomère polymérisable (A) contient un monomère polymérisable à base de dipentaérythritol.

**9.** Composition dentaire durcissable selon l'une quelconque des revendications 1 à 8, dans laquelle l'initiateur de polymérisation à base d'amine organique (B) contient un composé d'amine organique représenté par la formule (I) ou (II) suivante.

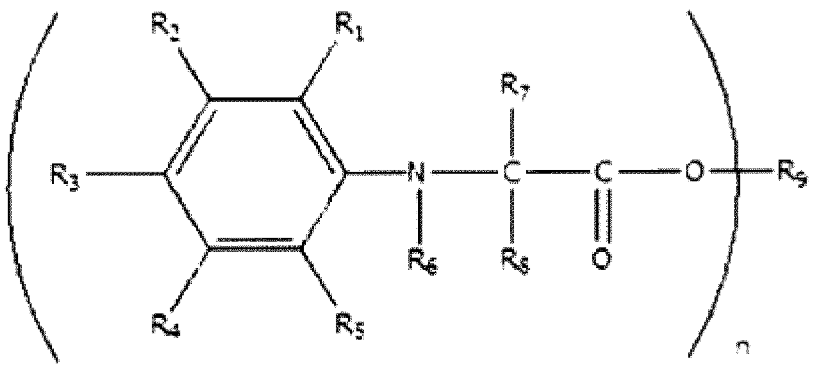

(I)

(dans laquelle $R_1$, $R_2$, $R_3$, $R_4$ et $R_5$ sont chacun indépendamment un atome d'hydrogène, un groupe alkyle, un

groupe aryle, un groupe aryloxy, un groupe alcoxyle, un groupe nitro, un groupe acyle, un groupe acyloxy, un groupe hydroxyle ou un atome d'halogène, $R_6$ est un atome d'hydrogène, un groupe alkyle ou un groupe aryle, $R_7$ et $R_8$ sont chacun indépendamment un atome d'hydrogène ou un groupe alkyle, $R_9$ est un atome d'hydrogène ou un atome de métal, et n est 1 quand $R_9$ est un hydrogène et le même nombre entier que la valence d'un atome de métal quand $R_9$ est l'atome de métal.)

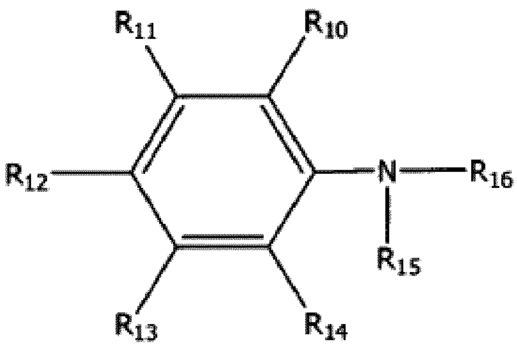

(II)

(dans laquelle $R_{10}$, $R_{11}$, $R_{12}$, $R_{13}$ et $R_{14}$ sont chacun indépendamment un atome d'hydrogène, un groupe alkyle, un groupe aryle, un groupe aryloxy, un groupe alcoxyle, un groupe nitro, un groupe acyle, un groupe acyloxy, un groupe hydroxyle ou un atome d'halogène, et $R_{15}$ et $R_{16}$ sont chacun indépendamment un atome d'hydrogène, un groupe alkyle, un groupe aryle ou un groupe alkyle substitué ayant un hétéroatome.)

**10.** Composition dentaire durcissable selon l'une quelconque des revendications 1 à 9, dans laquelle le matériau de résine souple (C) présente une dureté D d'après duromètre d'au plus 60.

**11.** Composition dentaire durcissable selon l'une quelconque des revendications 1 à 10 qui comprend en outre (D) un composé de métal de transition.

**12.** Composition dentaire durcissable selon la revendication 11, dans laquelle la teneur du composant (D) est de 0,001 à 10 parties en poids sur la base de 100 parties en poids du total des composants (A) et (B).

**13.** Composition dentaire durcissable selon l'une quelconque des revendications 1 à 12 qui comprend en outre (E) un composé réducteur contenant du soufre.

**14.** Composition dentaire durcissable selon la revendication 13, dans laquelle la teneur du composant (E) est de 0,01 à 10 parties en poids sur la base de 100 parties en poids du total des composants (A) et (B).

**15.** Composition dentaire durcissable selon l'une quelconque des revendications 1 à 14 qui comprend en outre (F) un composé d'acide hydroxylique.

**16.** Composition dentaire durcissable selon la revendication 15, dans laquelle la teneur du composé d'acide hydroxylique (F) est de 0,01 à 10 parties en poids sur la base de 100 parties en poids du total des composants (A) et (B).

**17.** Composition dentaire durcissable selon l'une quelconque des revendications 1 à 16 qui comprend en outre un peroxyde en une quantité d'au plus 0,1 partie en poids sur la base de 100 parties en poids du total des composants (A) et (B).

**18.** Composition dentaire durcissable selon la revendication 1, dans laquelle la teneur du solvant (G) est de 0,1 à 300 parties en poids sur la base de 100 parties en poids du total des composants (A) et (B).

**19.** Composition dentaire durcissable selon l'une quelconque des revendications 1 à 18 qui comprend en outre (H) au moins une charge sélectionnée parmi une charge inorganique et une charge composite organique.

**20.** Composition dentaire durcissable selon la revendication 19, dans laquelle la teneur de la charge (H) est de 1 à 400 parties en poids sur la base de 100 parties en poids du total des composants (A) et (B).

**21.** Composition dentaire durcissable selon la revendication 19, dans laquelle la charge inorganique (H) est au moins l'une sélectionnée dans le groupe constitué de l'oxyde de zirconium, de l'oxyde de zinc, du sulfate de baryum, de l'oxyde de bismuth, de l'oxychlorure de bismuth, de l'oxyde de carbonate de bismuth et du tungstate de calcium.

**22.** Composition dentaire durcissable selon l'une quelconque des revendications 1 à 21 qui comprend en outre (I) un agent de stérilisation.

**23.** Composition dentaire durcissable selon la revendication 22, dans laquelle l'agent de stérilisation (I) est au moins l'un sélectionné dans le groupe constitué du chlorure de benzalkonium, du chlorure de benzéthonium, de l'isopropylméthylphénol, du chlorure de cétylpyridinium, de la résorcine, du chlorhydrate de chlorhexidine, du gluconate de chlorhexidine, de l'iode, de l'iodure de potassium, de la povidone iodée et de l'iodoforme.

**24.** Composition dentaire durcissable selon la revendication 22 ou 23, dans laquelle la teneur de l'agent de stérilisation (I) est de 0,01 à 200 parties en poids sur la base de 100 parties en poids du total des composants (A) et (B).

**25.** Composition dentaire durcissable selon l'une quelconque des revendications 1 à 24 qui comprend en outre (J) un polymère autre que le composant (C) qui se dissout dans le composant (A) et/ou le composant (G).

**26.** Composition dentaire durcissable selon la revendication 25, dans laquelle la teneur du polymère (J) est de 0,1 à 50 parties en poids sur la base de 100 parties en poids du total des composants (A) et (B).

**27.** Composition dentaire durcissable selon la revendication 5, dans laquelle le monomère polymérisable à longue chaîne (A') est un di(méth)acrylate de polyéthylène glycol et/ou un di(méth)acrylate de polypropylène glycol ayant 11 à 28 motifs récurrents dérivés du propylène glycol et/ou de l'éthylène glycol.

**28.** Composition dentaire durcissable selon l'une quelconque des revendications 1 à 26 pour une utilisation en médecine dentaire comme matériau de remplissage de canal radiculaire, matériau de revêtement, matériau d'étanchéité temporaire ou matériau de scellement temporaire.

Fig. 1

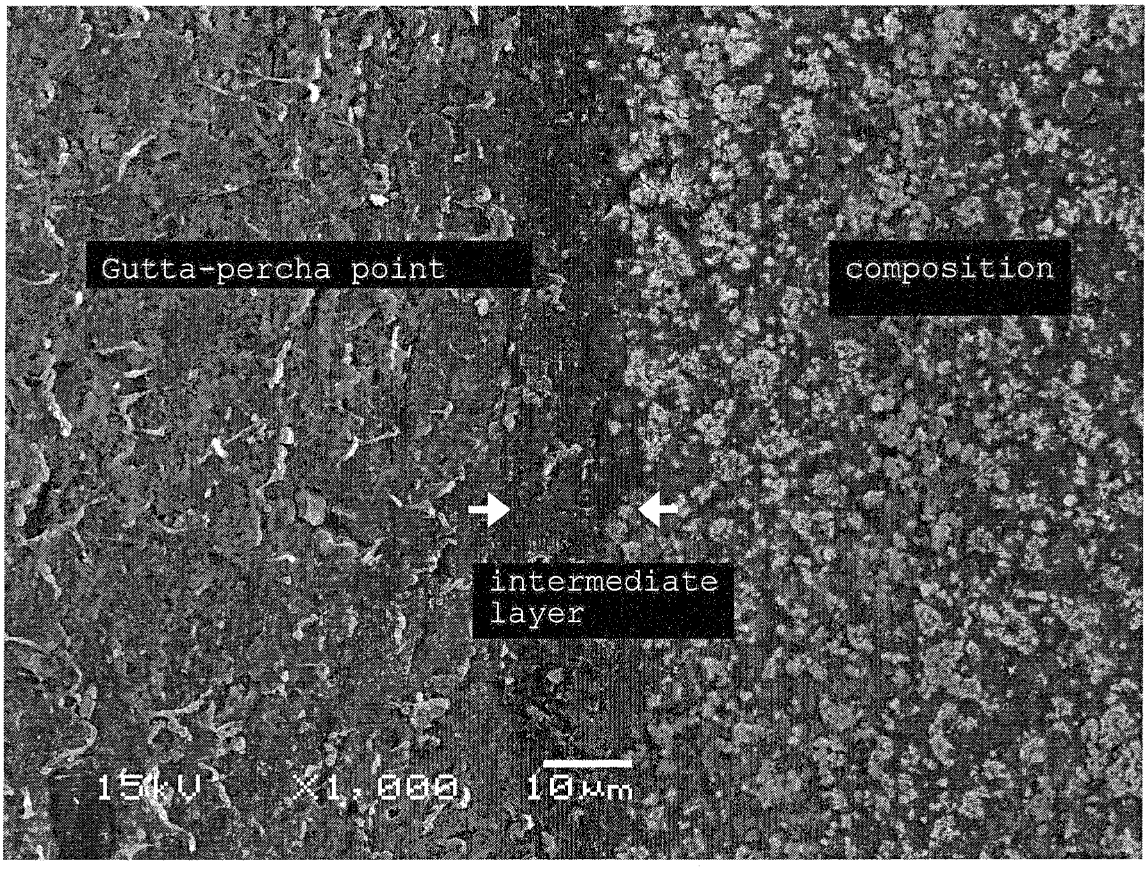
Gutta-percha point
composition
intermediate layer
15kV X1,000 10µm

Fig. 2

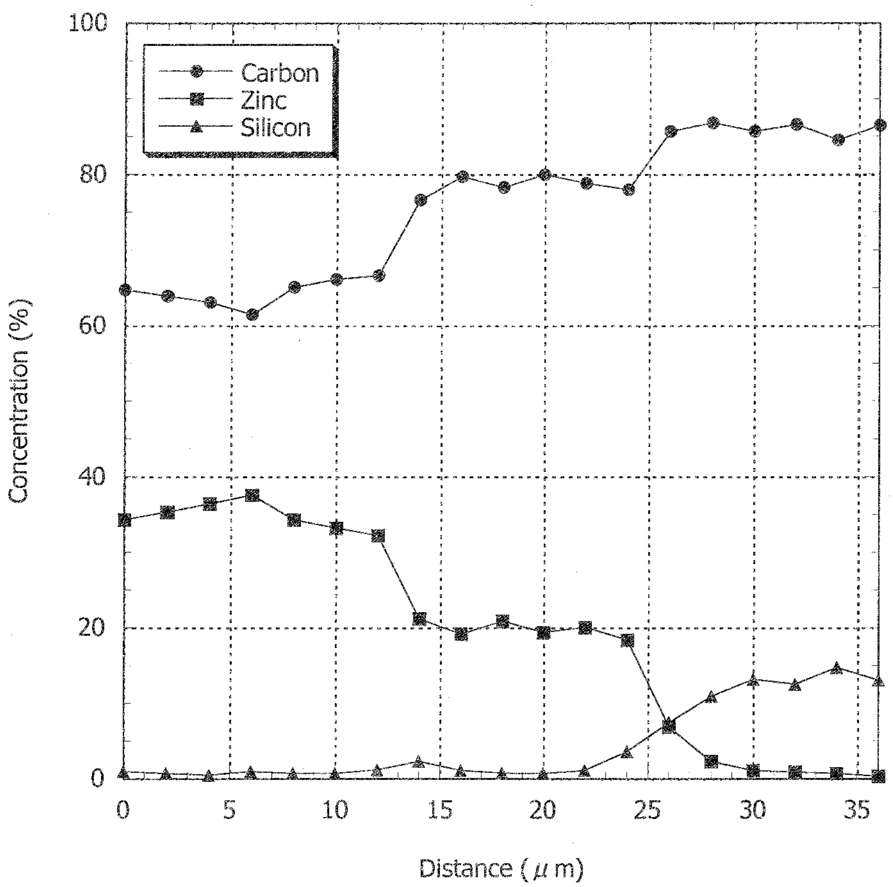
Carbon
Zinc
Silicon
Concentration (%)
Distance (μm)
0
20
40
60
80
100
0
5
10
15
20
25
30
35

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002187907 A
- JP 2003096122 A
- US 20020198284 A
- EP 1695685 A
- US 20090227699 A
- JP 2000143432 A
- JP 2008024668 A
- JP 2005179282 A
- JP 2008285645 A
- JP 2008308418 A
- JP S6317816 B